# EUROPEAN PATENT SPECIFICATION

(11) **EP 4 144 391 B1**
(45) Date of publication and mention of the grant of the patent: **24.06.2026**
(21) Application number: 20933928.2
(22) Date of filing: 28.04.2020
(51) Int. Cl.: A61M 5/24, A61M 5/315

(54) **SYRINGE**
SPRITZE
SERINGUE

(43) Date of publication of application: 08.03.2023
(73) Proprietor: CC Biotechnology Corporation, Tainan City, Taiwan 70955 (TW)
(72) Inventor: YEH, Chin-Min, Tainan City, Taiwan (TW)
(74) Representative: Savi, Massimiliano
(86) International application number: PCT/CN2020/087378
(87) International publication number: WO 2021/217390

(56) References cited:
- EP-A1- 2 452 711
- EP-A2- 0 937 471
- CN-A- 1 835 774
- CN-A- 101 829 381
- CN-A- 102 665 802
- CN-A- 102 716 529
- CN-A- 103 547 304
- CN-U- 203 090 111
- US-A- 5 938 642
- US-A1- 2006 206 057
- US-A1- 2013 131 605

## Description

### Field of the Invention

The present invention relates to a syringe, and especially to a syringe that can be assembled with a medicine vial to perform quantitative output of medicament liquid for multiple times, and can be replaced with another full medicine vial for reuse after medicament liquid in the medicine vial is completely used up.

### Description of Related Art

Based on the safety regulations for medicament liquid injection, a conventional syringe already has a function of being repeatedly used for a limited times. The conventional syringes are distinguished into a type of dose metering syringes and a type of frequency metering syringes. Regarding a conventional dose metering syringe, after the dose metering syringe is connected to a medicine vial, a pushing rod disposed at a rear end of the syringe is pushed, and a piston in the medicine vial is pushed to move for a predetermined distance via an interaction of a pushing mechanism mounted therein, thereby outputting a predetermined dose of medicament liquid through a needle connected to a front end of the medicine vial.

The conventional dose metering syringe is capable of achieving the function of outputting a predetermined dose of medicament liquid or further has a function of labor-saving in manipulation. However, after the medicament liquid in the medicine vial connected to the conventional dose metering syringe is used up, because the pushing mechanism in the syringe does not have a function to return to an original condition, all the conventional syringes need to be discarded and cannot be reused.

In addition, other conventional syringes are also disclosed in US 2006/206057 A1, US 2013/131605 A1, and CN 101 829 381 A.

### Content of the Invention

The technical problem to be solved by the present invention is: to provide a syringe to solve the problem that the conventional dose metering syringe cannot restore an original condition to be reused after being used.

The technical solution proposed by the present invention is: to provide a syringe detachably assembled with a medicine vial containing medicament liquid and a needle component connected to a front end of the medicine vial. Wherein the syringe defines a central axis extending along a front-to-rear direction of the syringe; the syringe comprises an injection mechanism. The injection mechanism has an injection mode and a standby mode, and is able to be manually triggered to perform a conversion between the injection mode and the standby mode. The injection mechanism comprises:
a housing having a movable space formed through the housing along the central axis, a front end of the housing detachably connected to the medicine vial; and a one-way ratchet portion formed on a peripheral wall of the movable space at a front segment of the movable space of the housing; and the housing having at least one spiral guiding rib formed on an inner peripheral wall of the housing;
a spiral pushing assembly mounted in the movable space of the housing and being capable of spirally moving around the central axis; the spiral pushing assembly extending to an outside of a rear end of the housing; and the spiral pushing assembly being able to be pushed to generate a spiral propulsion; the spiral pushing assembly comprising a driving spiral tube; the driving spiral tube mounted in and being capable of spirally moving in the movable space of the housing; the driving spiral tube having a driving spiral guiding groove formed in an external peripheral wall of the driving spiral tube and screwed with the at least one spiral guiding rib of the housing;
a metering guide sleeve mounted in the housing, being capable of rotating at a fixing point; and the metering guide sleeve inserted into the spiral pushing assembly and able to be controlled to connect and rotate with the spiral pushing assembly; the metering guide sleeve comprising a guide sleeve body and multiple non-return ratchet buckles; the guide sleeve body having a guide sleeve central hole, at least one spiral portion formed on an inner peripheral wall of the guide sleeve central hole; a guide sleeve threaded portion formed on a rear segment of an outer peripheral surface of the guide sleeve body; and a guide sleeve front end portion formed at a front end of the guide sleeve body; the multiple non-return ratchet buckles formed on an external surface of the guide sleeve front end portion; when the multiple non-return ratchet buckles controlled to engage with the one-way ratchet portion of the housing, the metering guide sleeve solely driven to rotate forwardly and in said injection mode; when the multiple non-return ratchet buckles disengaging from the one-way ratchet portion of the housing, the metering guide sleeve able to rotate forwardly or backwardly and in said standby mode;
a total volume control nut mounted in the spiral pushing assembly, being capable of linearly moving along the central axis, and screwed with the guide sleeve threaded portion at a rear segment of the metering guide sleeve;
a diagonal tension knob mounted in the front end of the housing and being movable along the central axis; the diagonal tension knob being drivable to change an engaging condition of the non-return ratchet buckles relative to the one-way ratchet portion of the housing and to convert the said injection mode and the said standby mode; and
a metering screw inserted in the diagonal tension knob and the housing and the metering screw screwed within the metering guide sleeve; the metering screw having a screw body having a screw threaded portion and the metering screw screwed with said spiral portion inside the metering guide sleeve via the screw threaded portion; a front end of the metering screw extending in front of the diagonal tension knob; when the injection mechanism in the injection mode, the diagonal tension knob triggered to move backward and to drive the multiple non-return ratchet buckles to engage with the one-way ratchet portion of the housing, a spiral movement of the metering guide sleeve able to drive the metering screw to move linearly along the central axis inside the housing to perform a quantitative output of the medicament liquid in the medicine vial; when the injection mechanism in the standby mode, the diagonal tension knob being able to move forward to make the multiple non-return ratchet buckles disengage from the one-way ratchet portion of the housing, the metering screw being able to linearly move backward and be received into the housing along the central axis.

According to the above-mentioned syringe, the syringe comprises a cartridge sleeve. The cartridge sleeve has a containing space formed through and within the cartridge sleeve along the central axis. The cartridge sleeve is detachably installed to the medicine vial, and a front end of the cartridge sleeve is detachably installed to the needle component. The cartridge sleeve is able to be connected to the front end of the housing of the injection mechanism. The cartridge sleeve is able to trigger the diagonal tension knob to move backward and to drive the multiple non-return ratchet buckles to engage with the one-way ratchet portion of the housing and in the said injection mode. When the cartridge sleeve is detached from the front end of the housing of the injection mechanism, the diagonal tension knob is able to move forward to make the multiple non-return ratchet buckles disengage from the one-way ratchet portion of the housing and in the said standby mode.

According to the above-mentioned syringe, the cartridge sleeve comprises a cartridge body. The cartridge body has at least one viewing window formed in a peripheral wall of the cartridge body. A connection portion is formed at a rear end of the cartridge body. A positioning flange is formed on the peripheral wall of the cartridge body and located at a front end of the connection portion. Multiple engaging protrusions are formed on an outer peripheral surface of the connection portion. The front end of the housing is a cartridge sleeve connecting portion. Multiple L-shaped engaging grooves are formed in an inner peripheral wall of the cartridge sleeve connecting portion. Each L-shaped engaging groove includes a straight groove section being parallel to the central axis and a transverse section connected to a rear end of the straight groove section. When the connection portion at the rear end of the cartridge sleeve is inserted into the cartridge sleeve connecting portion at the front end of the housing, each engaging protrusion is able to slide along the straight groove section of a respective one of the L-shaped engaging grooves and to turn into an end of the transverse section of the respective one of the L-shaped engaging grooves to lock the cartridge sleeve to the front end of the housing.

According to the above-mentioned syringe, a guiding ring is formed in the front end of the housing. An outer peripheral surface of the guiding ring is connected to a peripheral wall of the movable space via multiple arms. A spacing is formed between the guiding ring and the peripheral wall of the movable space. At least one guiding block is formed on an inner annular wall of the guiding ring. The screw body of the metering screw has at least one axial guiding groove passing through the screw threaded portion and being parallel to the central axis. The axial guiding groove engages with the guiding block inside the guiding ring of the housing.

According to the above-mentioned syringe, each non-return ratchet buckle has a wing protruding forwardly and disposed at a front end face of the non-return ratchet buckle. The diagonal tension knob has a conical surface abutting against the wing of each non-return ratchet buckle to let the conical surface of the diagonal tension knob push the non-return ratchet buckle to engage with the one-way ratchet portion of the housing via the wing.

According to the above-mentioned syringe, the syringe comprises a cartridge sleeve. The cartridge sleeve has a containing space formed through and within the cartridge sleeve along the central axis. The cartridge sleeve is detachably installed to the medicine vial, and a front end of the cartridge sleeve is detachably installed to the needle component. The cartridge sleeve is able to be connected to the front end of the housing of the injection mechanism. The cartridge sleeve is able to trigger the diagonal tension knob to move backward and to drive the multiple non-return ratchet buckles to engage with the one-way ratchet portion of the housing and in the said injection mode. When the cartridge sleeve is detached from the front end of the housing of the injection mechanism, the diagonal tension knob is able to move forward to make the multiple non-return ratchet buckles disengage from the one-way ratchet portion of the housing and in the said standby mode.

According to the above-mentioned syringe, the cartridge sleeve comprises a cartridge body. The cartridge body has at least one viewing window formed in a peripheral wall of the cartridge body. A connection portion is formed at a rear end of the cartridge body. A positioning flange is formed on the peripheral wall of the cartridge body and located at a front end of the connection portion. Multiple engaging protrusions are formed on an outer peripheral surface of the connection portion. The front end of the housing is a cartridge sleeve connecting portion. Multiple L-shaped engaging grooves are formed in an inner peripheral wall of the cartridge sleeve connecting portion. Each L-shaped engaging groove includes a straight groove section being parallel to the central axis and a transverse section connected to a rear end of the straight groove section. When the connection portion at the rear end of the cartridge sleeve is inserted into the cartridge sleeve connecting portion at the front end of the housing, each engaging protrusion is able to slide along the straight groove section of a respective one of the L-shaped engaging grooves and to turn into an end of the transverse section of the respective one of the L-shaped engaging grooves to lock the cartridge sleeve to the front end of the housing.

According to the above-mentioned syringe, the housing has at least one spiral guiding rib formed on an inner peripheral wall of the housing. The spiral pushing assembly comprises a clutch sleeve, and an output button. Wherein:
The driving spiral tube has a spiral tube channel formed through the driving spiral tube along the central axis, a guiding curved surface formed at a front segment of the spiral tube channel and converging forwardly along the central axis, and a spiral tube rear end portion disposed at a rear end of the driving spiral tube. The spiral tube rear end portion extends to an outside of the rear end of the housing and blocked by the rear end of the housing.

The clutch sleeve is mounted in the spiral tube channel of the driving spiral tube and is able to move linearly relative to the driving spiral tube. The clutch sleeve has a sleeve body, multiple flexible locking claws formed at a front end of the sleeve body, and a connecting end portion disposed at a rear end of the sleeve body. A sleeve channel is formed in the sleeve body and extending along the central axis. At least one sleeve guide groove portion is formed on a peripheral wall of the sleeve channel and is parallel to the central axis. The multiple flexible locking claws have flexibility for radial expansion and contraction. When the clutch sleeve is moved frontward relative to the driving spiral tube, the multiple flexible locking claws are abutted by the guiding curved surface at a front end of the driving spiral tube and contract radially; and the multiple flexible locking claws are capable of bouncing backward to an original position along the guiding curved surface and expand.

The output button is disposed at an outside behind the rear end of the housing and is connected with the connecting end portion at a rear end of the clutch sleeve.

The metering guide sleeve is inserted in the driving spiral tube and the clutch sleeve and is able to be clamped by the multiple flexible locking claws of the clutch sleeve.

The total volume control nut has at least one flange rib formed on an outer peripheral surface of the total volume control nut and engaging with said at least one sleeve guide groove portion of the clutch sleeve.

According to the above-mentioned syringe, each of the flexible locking claws has a locking surface formed at an inner surface of the flexible locking claw. The locking surface of each of the flexible locking claws has multiple straight stripes extending along a front-to-back direction. The metering guide sleeve has an engaging surface formed on the outer peripheral surface, located between the guide sleeve front end portion and the guide sleeve threaded portion, of the guide sleeve body. The engaging surface has multiple straight stripes being parallel to the central axis and corresponding to the straight stripes of the locking surfaces of the flexible locking claws for engagement.

According to the above-mentioned syringe, the housing has a scale observing window formed at a rear section of an external peripheral wall of the housing; the driving spiral tube has a spiral scale portion formed on the external peripheral wall of the driving spiral tube and being parallel to the driving spiral guiding groove; the spiral scale portion has multiple scale lines able to be observed through the scale observing window of the housing.

The beneficial effect that can be achieved by the present invention is that when the syringe is in use, the front end of the injection mechanism is connected to the medicine vial, with the whole arrangement of the syringe, the injection mechanism can be manually triggered to covert between the injection mode and the standby mode. Wherein, when the diagonal tension knob of the injection mechanism is triggered to move backward, the multiple non-return ratchet buckles are driven to engage with the one-way ratchet portion of the housing to be in the injection mode to provide a function of quantitatively outputting medicament liquid. After the medicament liquid in the medicine vial is used up, the medicine vial is removed, the injection mechanism can be manually triggered to move the diagonal tension knob frontward. Whereby, the non-return ratchet buckles are disengaged from the one-way ratchet portion of the housing to be in the standby mode. So the metering screw can be directly manually pushed back to the default position. The metering guide sleeve is driven to rotate backwardly at the same time. The total volume control nut is moved back to the initial full position of the metering guide sleeve. Accordingly, the syringe can be reused.

The syringe of the present invention can further use the cartridge as a triggering component to convert the injection mechanism between the injection mode and the standby mode. The cartridge has functions of installing the medicine vial and the needle component. When the cartridge is mounted to the front end of the housing of the injection mechanism, the diagonal tension knob is pushed to automatically move backward to drive the multiple non-return ratchet buckles to engage with the one-way ratchet portion of the housing to be in the injection mode. When the cartridge is detached from the front end of the housing of the injection mechanism, the diagonal tension knob can move forward to allow the non-return ratchet buckles to disengage from the one-way ratchet portion of the housing to be in the standby mode.

The syringe of the present invention may further have the guiding ring in the front end of the housing. The guiding ring is spaced from the peripheral wall of the movable space. The at least one guiding block is formed on the inner annular surface of the guiding ring. The screw body of the metering screw has an axial guiding groove passing through the screw threaded portion and being parallel to the central axis. The axial guiding groove is engaged with the guiding block in the guiding ring of the housing. Thus, when the medicament liquid in the medicine vial is used up, the user detaches the cartridge with the medicine vial from the front end of the housing, the non-return ratchet buckles of the metering guide sleeve are disengaged from the one-way ratchet portion of the housing to release the unidirectional restriction, and the axial guiding groove of the metering screw can be aligned with the guiding block in the guiding ring of the housing. So the metering screw extending out from the housing can be quickly and directly pushed back to position along the central axis to drive the metering guide sleeve to rotate backwardly and to move the total volume control nut back to the initial full position of the metering guide sleeve along the central axis, thereby enhancing convenience of use.

The spiral pushing assembly of the syringe of the present invention may further comprise the driving spiral tube, the clutch sleeve, and the output button. Wherein, the driving spiral tube is spirally movably mounted in the housing. The front end segment of the spiral tube channel is formed as the guiding curved surface. The clutch sleeve is spirally moved in the spiral tube channel of the driving spiral tube. The rear end of the clutch sleeve extends out from the rear end of the housing and is connected with the output button. The clutch sleeve has the multiple flexible locking claws formed on the front end of the sleeve body thereof. The multiple flexible locking claws have flexibility for radial expansion and contraction. When the clutch sleeve is moved forward relative to the driving spiral tube, the multiple flexible locking claws are abutted against the guiding curved surface at the front end of the driving spiral tube to be radially contracted to clamp the metering guide sleeve. So the metering guide sleeve, the clutch sleeve, and the driving spiral tube can be rotated together. The multiple flexible locking claws can spring back to position along the guiding curved surface to expansion to release the metering guide sleeve. Accordingly, the metering guide sleeve is smoothly and reliably engaged with and disengaged from the metering guide sleeve.

The syringe of the present invention may further have the scale observing window formed at the external peripheral wall of the housing. The driving spiral tube has a spiral scale portion formed on the external peripheral wallof the driving spiral tube and being parallel to the driving spiral guiding groove. The spiral scale portion has multiple scale lines observed through the scale observing window of the housing. So the driving spiral tube as a single component have functions of driving and dose scale display.

### Brief Description of the Drawings

The following drawings are only intended to illustrate and explain the present invention, and do not limit the scope of the present invention. Wherein:
Fig. 1 is an exploded perspective view of a preferable embodiment of a syringe in accordance with the present invention and a medicine vial and a needle component, showing that the syringe uses a cartridge sleeve as a triggering component.
Fig. 2 is a cross sectional side view of the preferable embodiment of the syringe in Fig. 1 showing that the cartridge sleeve is connected to the medicine vial and is disconnected from an injection mechanism.
Fig. 3 is an exploded perspective view of the injection mechanism in Figs. 1 and 2.
Fig. 4 is another exploded perspective view of the injection mechanism in Figs. 1 and 2.
Fig. 5 is a front view of a housing of the injection mechanism in Figs. 3 and 4.
Fig. 6 is a front view of the injection mechanism in Figs. 3 and 4.
Fig. 7 is a cross sectional side view along line A-A in Fig. 6.
Fig. 8 is a cross sectional side view along line B-B in Fig. 6.
Fig. 9 is a perspective view of the injection mechanism in Figs. 3 and 4, wherein the housing is removed.
Fig. 10 is another perspective view of the injection mechanism in Figs. 3 and 4, wherein the housing is removed.
Fig. 11 is a partial perspective view of a clutch sleeve of the injection mechanism in Figs. 3 and 4 showing the clutch sleeve assembled with a metering guide sleeve.
Fig. 12 is a cross sectional front view of the housing of the injection mechanism in Figs. 3 and 4 showing the clutch sleeve assembled with the metering guide sleeve.
Fig. 13 is an operational cross sectional side view of the preferable embodiment of the syringe in Fig. 1 showing that the cartridge sleeve is connected to the medicine vial and is disconnected from the injection mechanism.
Fig. 14 is an operational cross sectional side view of the preferable embodiment of the syringe in Fig. 13 showing that the cartridge sleeve connected with the medicine vial and the needle component is assembled to a front end of the injection mechanism.
Fig. 15 is an operational cross sectional side view of the injection mechanism in Fig. 14, showing that a spiral pushing assembly of the injection mechanism is pulled out from a rear end of the housing.
Fig. 16 is an operational cross sectional side view of the injection mechanism in Fig. 15, showing that the spiral pushing assembly of the injection mechanism is pushed frontward to output medicament liquid for injection.
Fig. 17 is an operational cross sectional side view of the injection mechanism in Fig. 16, shown after one time injection.
Fig. 18 is an operational cross sectional side view of the injection mechanism in Fig. 17, showing that the spiral pushing assembly of the injection mechanism is pulled out from the rear end of the housing again.
Fig. 19 is an operational cross sectional side view of the syringe in accordance with the present invention showing that the medicament liquid in the medicine vial is used up.
Fig. 20 is an operational cross sectional side view of the syringe in Fig. 19 showing that the empty medicine vial and the cartridge sleeve around the medicine vial are detached from the front end of the injection mechanism.
Fig. 21 is an operational cross sectional side view of the injection mechanism in Fig. 20 showing that the dose metering spiral rod of the metering screw is pushed back to make the injection mechanism return to a start condition.

### Description of the Reference Numbers:

A central axis; 1 cartridge sleeve; 10 cartridge body; 100 containing space; 11 needle connecting portion; 12 connection portion; 13 positioning flange; 14 viewing window; 15 engaging protrusion; 2 injection mechanism; 20 housing; 200 movable space; 21 cartridge sleeve connecting portion; 211 L-shaped engaging groove; 22 guiding ring; 221 guiding block; 23 one-way ratchet portion; 24 arm; 25 spiral guiding rib; 26 scale observing window; 2A spiral pushing assembly; 30 driving spiral tube; 31 spiral tube channel; 32 guiding curved surface; 33 driving spiral guiding groove; 34 spiral tube rear end portion; 35 spiral scale portion; 40 clutch sleeve; 41 sleeve body; 42 flexible locking claw; 421 locking surface; 43 connecting end portion; 44 sleeve channel; 45 sleeve guide groove portion; 50 output button; 60 metering guide sleeve; 61 guide sleeve body; 62 non-return ratchet buckle; 621 wing; 63 guide sleeve central hole; 64 guide sleeve front end portion; 65 spiral portion; 66 guide sleeve threaded portion; 67 engaging surface; 70 total volume control nut; 71 flange rib; 80 diagonal tension knob; 81 inner hole; 82 conical surface; 90 metering screw; 91 screw body; 92 screw front end portion; 93 screw threaded portion; 94 axial guiding groove; 3 medicine vial; 3A needle component; 3B needle cap; 3C piston; 4 protection cap.

### Description of Present Invention

The following describes the technical means adapted by the present invention to achieve the intended purpose of the invention in conjunction with the drawings and the preferable embodiments of the present invention.

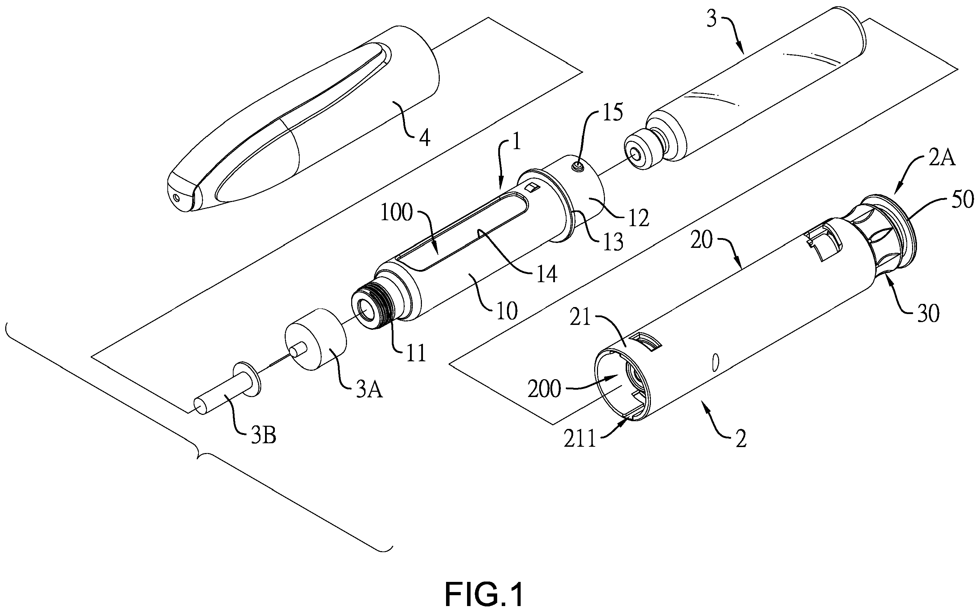

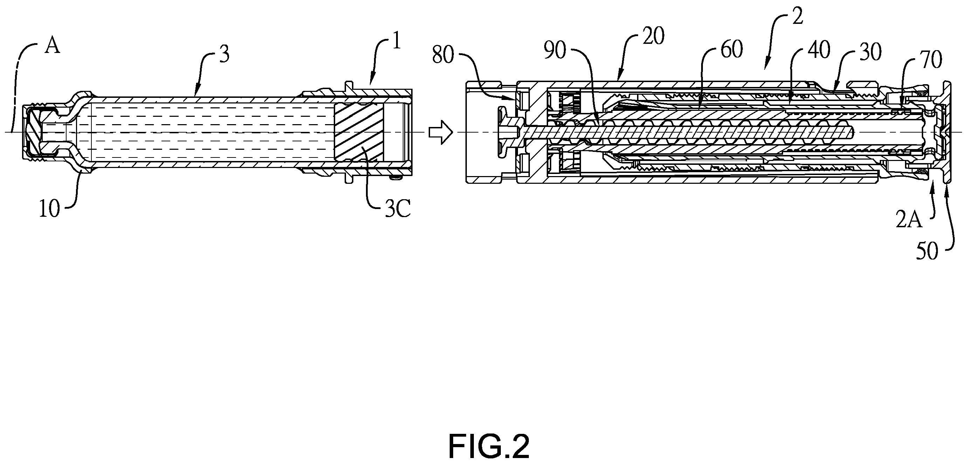

With reference to Figs. 1 and 2, according to the content of the invention disclosed above, the syringe in accordance with the present invention is able to be assembled with a medicine vial 3 containing medicament liquid and a needle component 3A connected to a front end of the medicine vial 3 to provide a device adapted to perform function of medicament liquid injection. The medicine vial 3 and the needle component 3A are both conventional components. The medicine vial 3 contains the medicament liquid and has a piston 3C mounted therein for pushing and outputting the medicament liquid.

As shown in Figs. 1 and 2 the syringe in accordance with the present invention mainly comprises an injection mechanism 2. The injection mechanism 2 has an injection mode and a standby mode. The injection mechanism 2 can be directly manually triggered through a user's hands or indirectly manually triggered through a triggering component to perform a conversion between the injection mode and the standby mode.

In the preferable embodiment shown in Figs. 1 and 2, the syringe uses a cartridge sleeve 1 as a triggering component to convert the modes of the injection mechanism 2. The injection mechanism 2 is triggered to covert from the standby mode to the injection mode via connecting the cartridge sleeve 1 to the front end of the injection mechanism 2. When the cartridge sleeve 1 is detached from the front end of the injection mechanism 2, the injection mechanism 2 automatically converts to the standby mode. However, the triggering component to convert the modes of the injection mechanism 2 is not limited to the cartridge sleeve 1 in the syringe of the present invention.

For convenience of illustration of the specific structure of the syringe of the present invention, the preferable embodiment shown in Figs. 1 and 2 is taken as an example for illustration. The syringe of the present invention defines a central axis A extending along a front-to-back direction of the syringe. The injection mechanism 2 comprises a housing 20, a spiral pushing assembly 2A, a metering guide sleeve 60, a total volume control nut 70, a diagonal tension knob 80, and a metering screw 90. The structure of each of the aforementioned components is illustrated below.

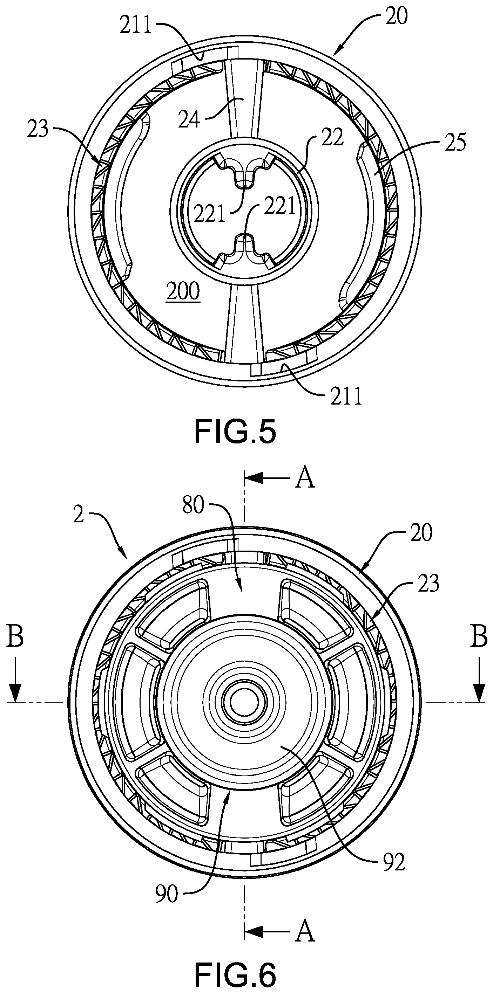

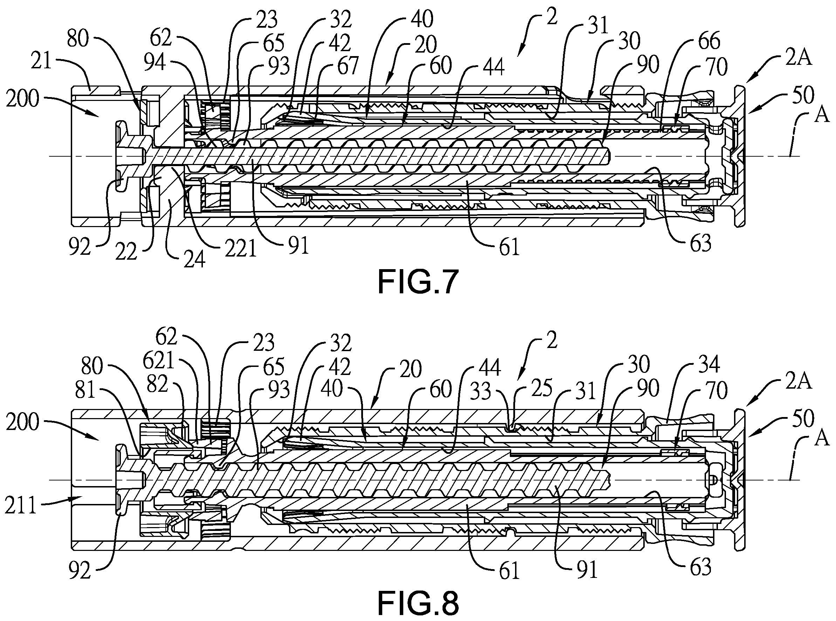

As shown in Figs. 1 to 5 and 6 to 8, the housing 20 has a movable space 200 formed through the housing 20 along the central axis A. A one-way ratchet portion 23 is formed inside the movable space 200 of the housing 20. The one-way ratchet portion 23 has multiple unidirectional angled ratchet teeth. The multiple unidirectional angled ratchet teeth are in a rotational and symmetric arrangement with respect to a predetermined forward direction of the central axis A. The front end of the housing 20 is detachably connected to the medicine vial 3.

As shown in Figs. 1 to 5 and 6 to 8, the housing 20 has a guiding ring 22 formed in the movable space 200 and located in front of the one-way ratchet portion 23. The guiding ring 22 is spaced from a peripheral wall of the movable space 200. In the preferable embodiment, an outer peripheral surface of the guiding ring 22 and the peripheral wall of the movable space 200 are connected to each other via multiple arms 24, so the guiding ring 22 is kept spaced from the peripheral wall of the movable space 200 by a predetermined distance. At least one guiding block 221 is formed on an inner annular wall of the guiding ring 22. In the preferable embodiment, the inner annular wall of the guiding ring 22 has two guiding blocks 221 being radially symmetric with respect to the central axis A. The housing 20 has one or multiple spiral guiding ribs 25 formed in the movable space 200 and at a rear segment of the inner peripheral wall of the housing 20.

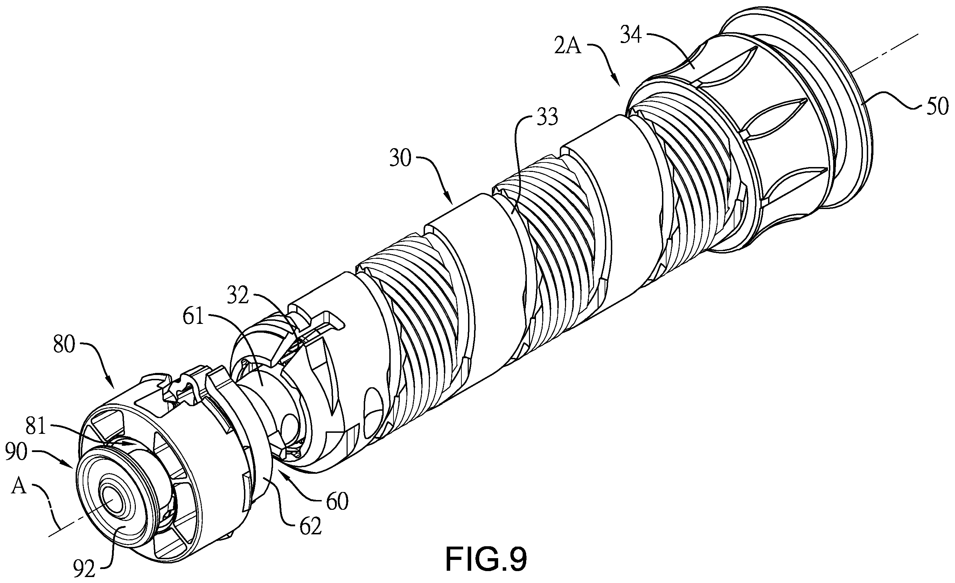

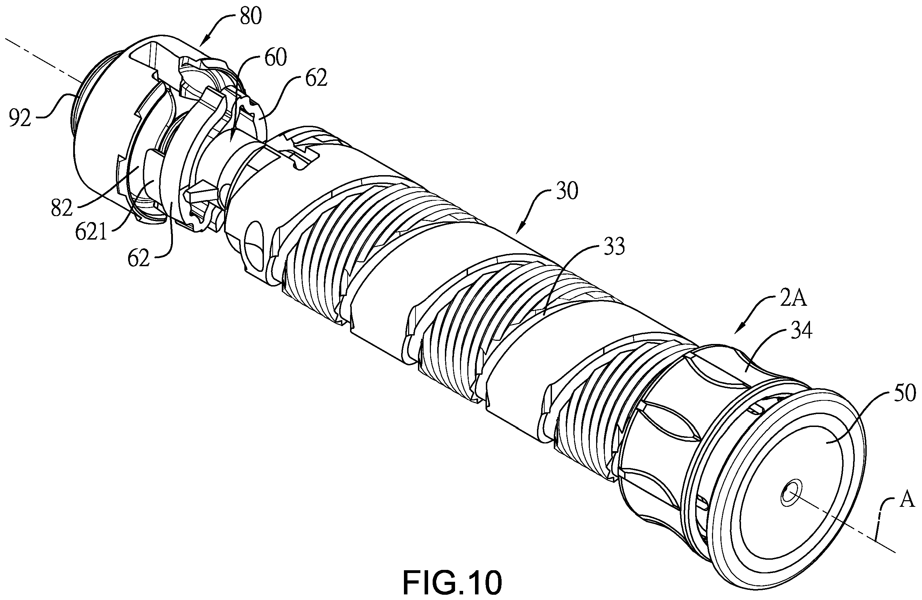

As shown in Figs. 2 to 4 and 6 to 10, the spiral pushing assembly 2A is mounted in the movable space 200 of the housing 20 and is capable of spirally moving around the central axis A. The spiral pushing assembly 2A extends out from a rear end of the housing 20 and is able to be pushed to generate a spiral propulsion. In the preferable embodiment, the spiral pushing assembly 2A comprises a driving spiral tube 30, a clutch sleeve 40, and an output button 50.

As shown in Figs. 2 to 4 and 6 to 10, the driving spiral tube 30 is mounted in the movable space 200 of the housing 20 and is capable of spirally moving. The driving spiral tube 30 has a spiral tube channel 31 formed through the driving spiral tube 30 along the central axis A. A guiding curved surface 32 is formed at a front segment of the spiral tube channel 31 and converges forwardly along the central axis A. That is, the spiral tube channel 31 at the front segment of the driving spiral tube 30 is formed as a curved surface having an opening gradually decreased from the rear to the front thereof. A driving spiral guiding groove 33 is formed in an external peripheral wall of the driving spiral tube 30. The pitch of the driving spiral guiding groove 33 is set according to the volume of the predetermined dose of the medicament liquid of the syringe. And the driving spiral guiding groove 33 is screwed with the spiral guiding ribs 25 in the housing 20. The driving spiral tube 30 has a spiral tube rear end portion 34 with a larger diameter and disposed at a rear end of the driving spiral tube 30. The spiral tube rear end portion 34 extends out from the rear end of the housing 20 and can be blocked by the rear end of the housing 20 to limit its moving path.

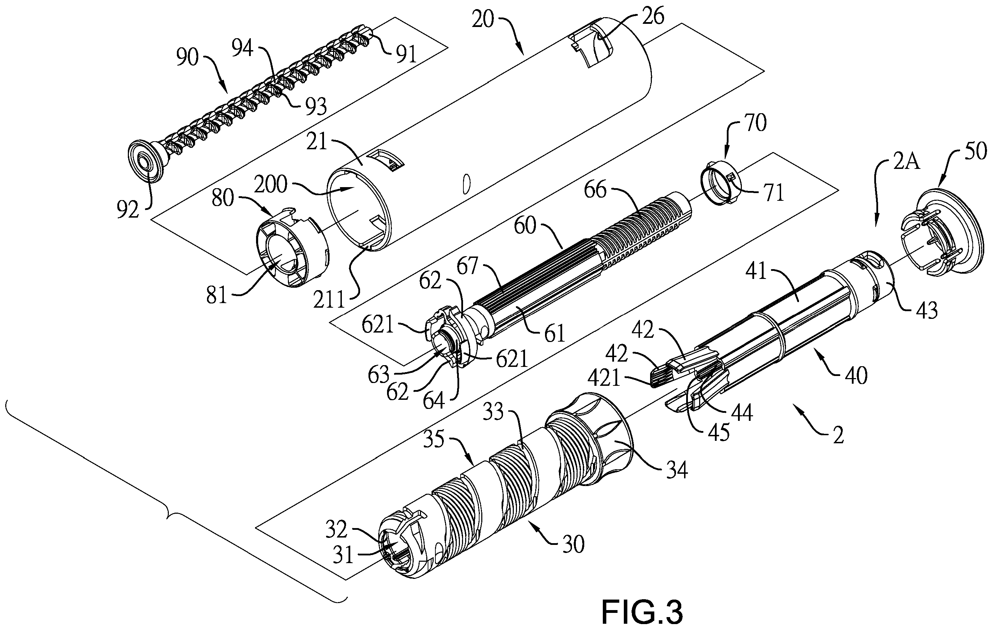

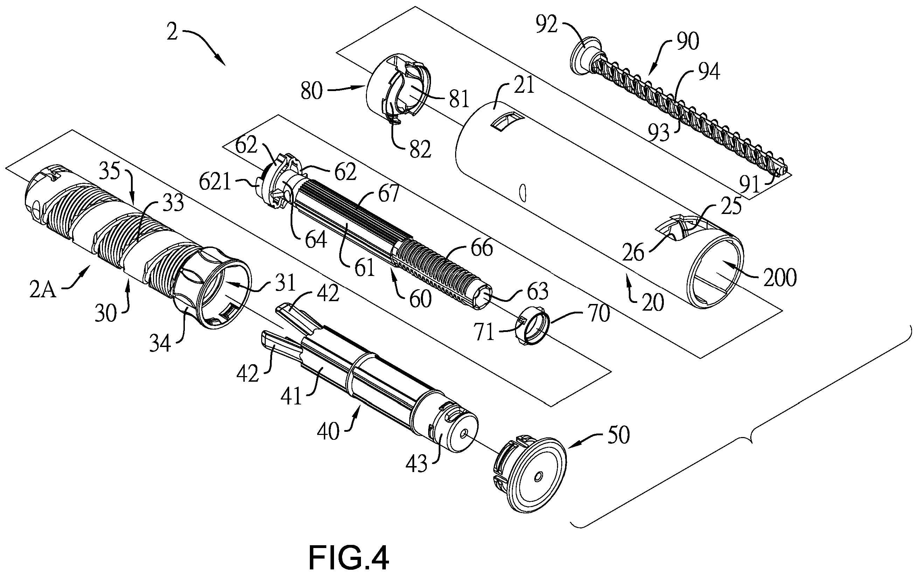

As shown in Figs. 3 and 4, a scale observing window 26 is formed through an external peripheral wall of the housing 20. A spiral scale portion 35 is formed on the external peripheral wall of the driving spiral tube 30 and is parallel to the driving spiral guiding groove 33. The spiral scale portion 35 has multiple scale lines (not shown in the drawings). The scale lines of the spiral scale portion 35 can be observed through the scale observing window 26 of the housing 20 and make the driving spiral tube 30 have functions of driving and dose scale display.

As shown in Figs. 2 to 4 and 6 to 8, the clutch sleeve 40 is mounted in the spiral tube channel 31 of the driving spiral tube 30 and able to linearly move relative to the driving spiral tube 30. The clutch sleeve 40 has a sleeve body 41, multiple flexible locking claws 42 formed at a front end of the sleeve body 41, and a connecting end portion 43 formed at a rear end of the sleeve body 41. A sleeve channel 44 is formed in the sleeve body 41 and forwardly extends along the central axis A. At least one sleeve guide groove portion 45 is formed on a peripheral wall of the sleeve channel 44 and is parallel to the central axis. The multiple flexible locking claws 42 have flexibility for radial expansion and contraction. When the clutch sleeve 40 moves frontward relative to the driving spiral tube 30, the multiple flexible locking claws 42 are abutted against the guiding curved surface 32 at a front end of the driving spiral tube 30 to radially contract and are capable of bouncing backward to an original position along the guiding curved surface 32 to expand. In the preferable embodiment, each flexible locking claw 42 has a locking surface 421 disposed at an inner surface of the flexible locking claw 42. The locking surface 421 has multiple straight stripes extending along the front-to-back direction.

The output button 50 is disposed outside the rear end of the housing 20 and is pivotally connected with the connecting end portion 43 at a rear end of the clutch sleeve 40. The output button 50 is adapted to provide an operational component for the user.

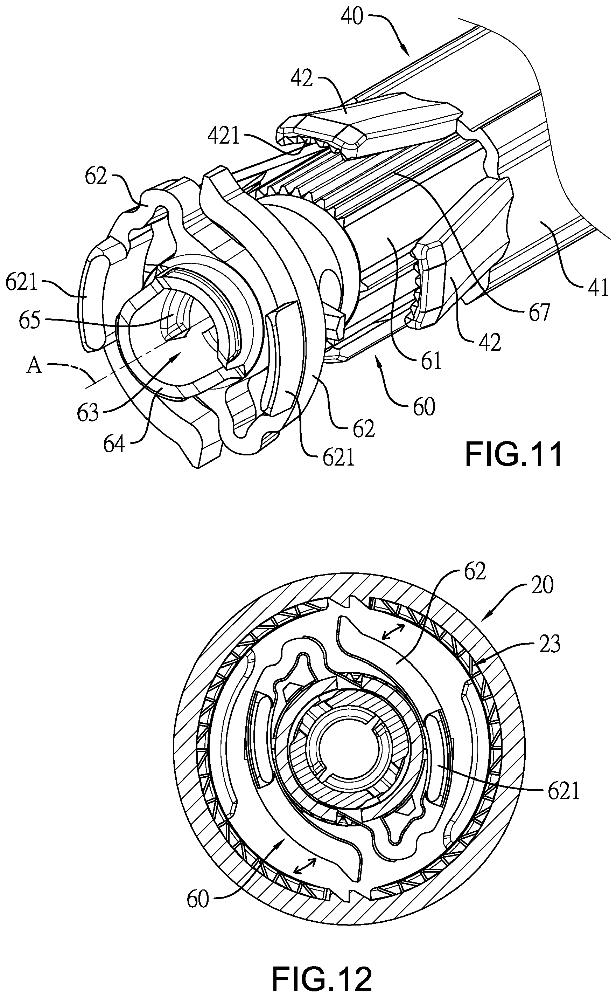

As shown in Figs. 2 to 4 and 6 to 11, the metering guide sleeve 60 is mounted in the housing 20, is capable of rotating at a fixing point, is inserted in the driving spiral tube 30 and the clutch sleeve 40, and is selectively clamped by the multiple flexible locking claws 42 of the clutch sleeve 40. When the flexible locking claws 42 of the clutch sleeve 40 are driven to contract by the guiding curved surface 32 of the driving spiral tube 30 to clamp the metering guide sleeve 60, the metering guide sleeve 60, the clutch sleeve 40, and the driving spiral tube 30 are able to rotate together. The metering guide sleeve 60 comprises a guide sleeve body 61 and multiple non-return ratchet buckles 62. The guide sleeve body 61 has a guide sleeve central hole 63 formed through the guide sleeve body 61 along the central axis A. The guide sleeve body 61 has a guide sleeve front end portion 64 formed at a front end of the guide sleeve body 61. The guide sleeve front end portion 64 is pivotally connected within the guiding ring 22 of the housing 20. At least one spiral portion 65 is formed on an inner peripheral wall of the guide sleeve central hole 63 at a segment thereof with respect to the guide sleeve front end portion 64. A guide sleeve threaded portion 66 is formed on a rear segment of an outer peripheral surface of the guide sleeve body 61. The guide sleeve threaded portion 66 has a pitch being smaller than the pitch of the driving spiral guiding groove 33 of the driving spiral tube 30. An engaging surface 67 is formed on the outer peripheral surface of the guide sleeve body 61 and is located between the guide sleeve front end portion 64 and the guide sleeve threaded portion 66. The engaging surface 67 has multiple straight stripes being parallel to the central axis A and corresponding to the straight stripes formed in the locking surfaces 421 of the flexible locking claws 42 for engagement. The multiple non-return ratchet buckles 62 are formed on an external surface of the guide sleeve front end portion 64. The multiple non-return ratchet buckles 62 are all curved and are in a rotational symmetric arrangement with respect to the forward direction of the central axis A. The multiple non-return ratchet buckles 62 can be controlled to engage with or disengage from the one-way ratchet portion 23 of the housing 20. When the non-return ratchet buckles 62 engage with the one-way ratchet portion 23 of the housing 20, the metering guide sleeve 60 can only rotate forwardly and cannot rotate backwardly, that is, in the injection mode. When the non-return ratchet buckles 62 disengage from the one-way ratchet portion 23 of the housing 20, the non-return function is released, so the metering guide sleeve 60 can freely rotate forwardly or backwardly, that is, in the standby mode. In the preferable embodiment, each non-return ratchet buckle 62 has a wing 621 protruding forwardly from a front end surface thereof.

As shown in Figs. 2 to 4 and 6 to 8, the total volume control nut 70 is mounted in the spiral tube channel 31 of the driving spiral tube 30, is capable of linearly moving along the central axis A, and is screwed with the metering guide sleeve 60. A total movable distance of the total volume control nut 70 relative to the guide sleeve threaded portion 66 of the metering guide sleeve 60 corresponds to a movable distance of the piston 3C in the medicine vial 3 to control a total output volume of the medicament liquid in the medicine vial 3. In the preferable embodiment, the total volume control nut 70 has an inner threaded hole and is screwed with the guide sleeve threaded portion 66 at the rear segment of the guide sleeve body 61 of the metering guide sleeve 60. The total volume control nut 70 has flange ribs 71 formed on an outer peripheral surface thereof. The flange ribs 71 engage with the sleeve guide groove portion 45 in the sleeve channel 44 of the clutch sleeve 40.

As shown in Figs. 2 to 4 and 6 to 10, the diagonal tension knob 80 is mounted in the front end of the housing 20 and is movable along the central axis A. The diagonal tension knob 80 can be driven to convert the engagement and the disengagement between the non-return ratchet buckles 62 and the one-way ratchet portion 23 of the housing 20 for conversion between the injection mode and the standby mode.

In the preferable embodiment, the diagonal tension knob 80 is mounted in the cartridge sleeve connecting portion 21 at the front end of the housing 20, through the space between the peripheral wall of the movable space 200 and the outer annular surface of the guiding ring 22, and has an inner hole 81 formed through the diagonal tension knob 80 along the central axis A. The diagonal tension knob 80 has a conical surface 82 having diameters gradually increasing forwardly. A rear section of the conical surface 82 abuts the wings 621 of the non-return ratchet buckles 62. When the diagonal tension knob 80 is forced to move backward, the diagonal tension knob 80 can drive the non-return ratchet buckles 62 of the metering guide sleeve 60 to expand radially by a radial and diagonal pushing force of the conical surface 82, and the non-return ratchet buckles 62 and one-way ratchet portion 23 of the housing 20 engage with each other. When the diagonal tension knob 80 is force free, the non-return ratchet buckles 62 contract through their own elasticity to disengage from the one-way ratchet portion 23 of the housing 20 and push the conical surface 82 of the diagonal tension knob 80 and make the diagonal tension knob 80 move forwardly to restore.

As shown in Figs. 2 to 4 and 6 to 10, the metering screw 90 is inserted in the diagonal tension knob 80 and the guiding ring 22 of the housing 20. The metering screw 90 is screwed within the metering guide sleeve 60 and is able to be driven to move linearly along the central axis A. The metering screw 90 comprises a screw body 91 and a screw front end portion 92 formed at a front end of the screw body 91. A screw threaded portion 93 is formed on an outer peripheral surface of the screw body 91 and has at least one axial guiding groove 94 being parallel to the central axis A and passing through the screw threaded portion 93. The screw threaded portion 93 is screwed with the spiral portion 65 inside the guide sleeve front end portion 64 of the metering guide sleeve 60. Said axial guiding grooves 94 are engaged with the guiding blocks 221 inside the guiding ring 22 of the housing 20. The screw front end portion 92 is located in front of the guiding ring 22 of the housing 20 and in front of the diagonal tension knob 80. A spiral movement of the metering guide sleeve 60 drives the metering screw 90 in the housing 20 to move linearly along the central axis A to push the piston 3C in the medicine vial 3 to preform quantitative output of the medicament liquid in the medicine vial 3. When the injection mechanism 2 is in the standby mode, the metering screw 90 extending out from the front end of the housing 20 can linearly move along the central axis A backwardly and be received into the housing 20.

When the syringe of the present invention uses the cartridge sleeve 1 as a triggering component to convert the modes of the injection mechanism 2, as shown in Figs. 1 and 2, the cartridge sleeve 1 contains the medicine vial 3 and the needle component 3A assembled with the medicine vial 3 and is detachably connected to the injection mechanism 2. In the preferable embodiment, the cartridge sleeve 1 has a containing space 100 formed therethrough along the central axis A. And the cartridge sleeve 1 has a cartridge body 10. The cartridge body 10 has a needle connecting portion 11 formed at a front end of the cartridge body 10 and a connection portion 12 formed at a rear end of the cartridge body 10. The containing space 100 has two openings respectively formed through the front end and the rear end of the cartridge sleeve 1. The medicine vial 3 is detachably mounted and fixed in the containing space 100 of the cartridge sleeve 1. The needle component 3A is detachably sleeved on the needle connecting portion 11. The connection portion 12 at the rear end of the cartridge body 10 is detachably connected to the injection mechanism 2.

As shown in Figs. 1 to 5 and 6 to 8, in the preferable embodiment, the needle connecting portion 11 has a thread formed on an outer peripheral surface thereof. The needle component 3A is screwed with the needle connecting portion 11. The cartridge body 10 has a positioning flange 13 formed on a peripheral wall thereof and located at a front end of the connection portion 12. One or multiple viewing windows 14 may be formed in the peripheral wall of the cartridge body 10 and communicate with the containing space100 to allow a user to observe the volume of the remaining medicament liquid in the medicine vial 3 in the cartridge sleeve 1.

As shown in Figs. 1 to 5 and 6 to 8, the front end of the housing 20 has a cartridge sleeve connecting portion 21. The connection portion 12 at the rear end of the cartridge sleeve 1 is detachably connected to the cartridge sleeve connecting portion 21 of the housing 20. The connecting structures between the cartridge sleeve connecting portion 21 at the front end of the housing 20 and the connection portion 12 at the rear end of the cartridge sleeve 1 can be engagement structures or threaded connection structures. In the preferable embodiment, the connecting structures between the cartridge sleeve connecting portion 21 at the front end of the housing 20 and the connection portion 12 at the rear end of the cartridge sleeve 1 is an engagement structure. Wherein, multiple engaging protrusions 15 are formed on an outer peripheral surface of the connection portion 12 at the rear end of the cartridge sleeve 1. Multiple L-shaped engaging grooves 211 are formed in an inner peripheral wall of the cartridge sleeve connecting portion 21 at the front end of the housing 20. Each L-shaped engaging groove 211 includes a straight groove section being parallel to the central axis A and a transverse section connected to a rear end of the straight groove section. To place the connection portion 12 at the rear end of the cartridge sleeve 1 into the cartridge sleeve connecting portion 21 at the front end of the housing 20, each engaging protrusion 15 slides along the straight groove section of the L-shaped engaging groove 211, then the cartridge sleeve 1 is turned in an angle to move the engaging protrusion 15 to fix and engage with an end of the transverse section of the L-shaped engaging groove 211, hereby locking the cartridge sleeve 1 to the front end of the housing 20.

As shown in Fig. 1, the syringe may further comprise a protection cap 4. The protection cap 4 is able to be sleeved on and surround the cartridge sleeve 1 assembled with the medicine vial 3 and the needle component 3A to protect the medicine vial 3 assembled with the needle component. In addition, a needle cap 3B may be sleeved on the needle component 3A to prevent the needle from being exposed and to ensure safety of use.

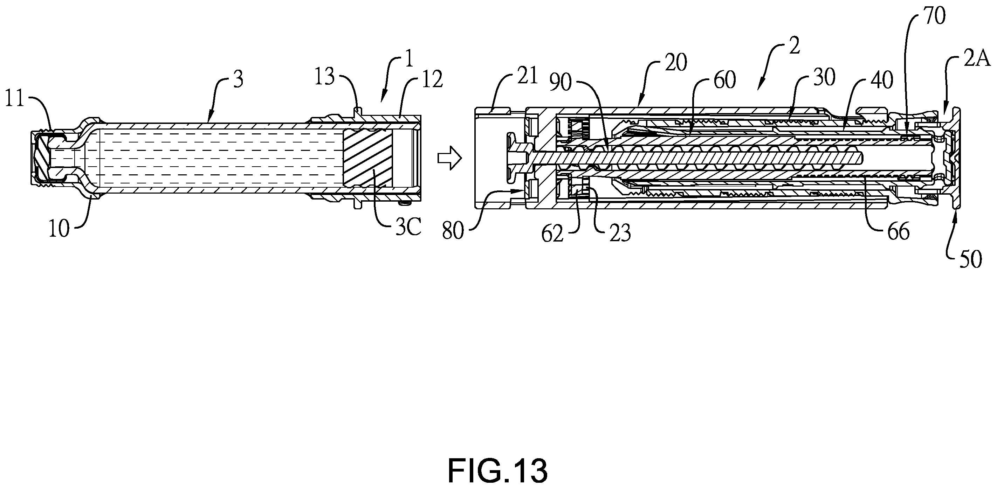

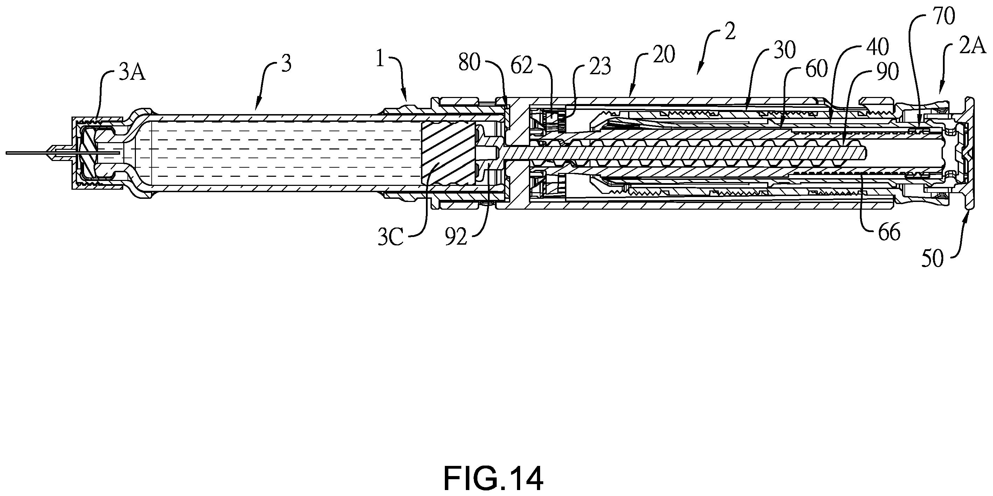

Regarding the condition of use of the syringe of the present invention, take the preferable embodiment of the syringe shown in Figs. 1 and 2 as an example. With reference to Figs. 13, 14 and Figs. 6 to 8, and Fig. 12, after the medicine vial 3 filled up with the medicament liquid is installed in the cartridge sleeve 1, the connection portion 12 at the rear end of the cartridge sleeve 1 is placed into the cartridge sleeve connecting portion 21 at the front end of the housing 20 of the injection mechanism 2 for assembly and is limited in position via the positioning flange 13 of the cartridge sleeve 1 abutting against the front end of the housing 20. At the same time, the diagonal tension knob 80 is pushed by the rear end of the cartridge sleeve 1 and moves backward. The non-return ratchet buckles 62 of the metering guide sleeve 60 are abutted by the conical surface 82 of the diagonal tension knob 80 to radially expand to engage with the one-way ratchet portion 23 of the housing 20. Whereby the injection mechanism 2 is in the injection mode, and the syringe is in a dose metering mode.

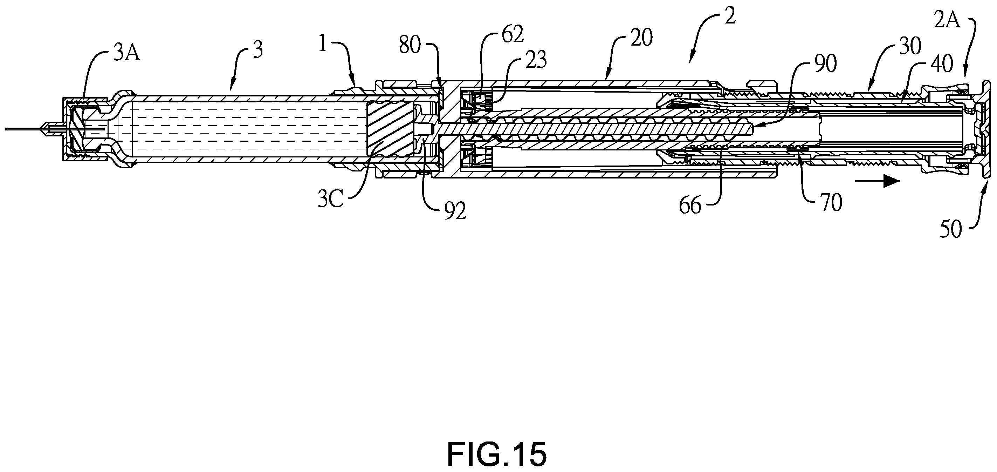

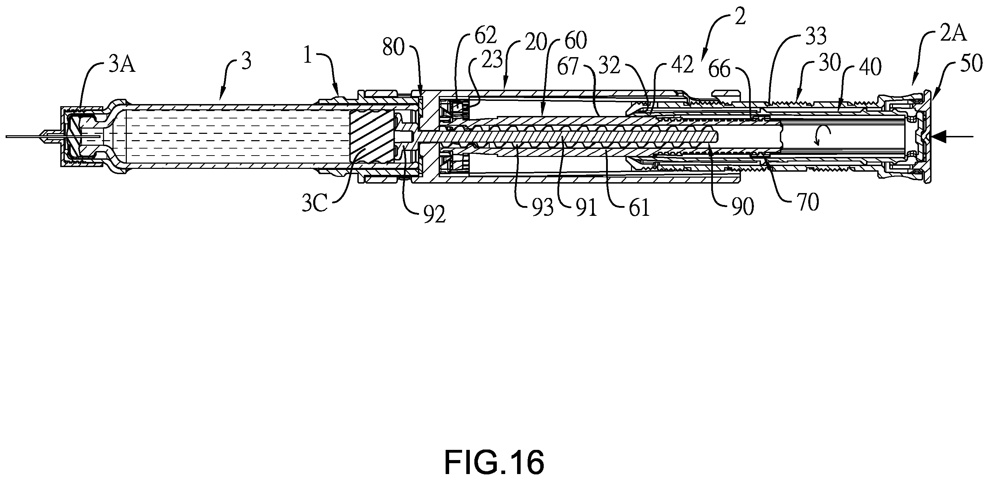

As shown in Figs. 15, 16 and 6 to 8, when the needle component 3A is installed at the front end of the cartridge sleeve 1, the medicine injection for a human body is able to be performed. Wherein, the output button 50 at the rear end of the spiral pushing assembly 2A is pushed by the user and the clutch sleeve 40 is pushed by the output button 50 and moves forwardly. When the flexible locking claws 42 at the front end of the clutch sleeve 40 are pushed by the guiding curved surface 32 at the front end of the driving spiral tube 30, the flexible locking claws 42 contract to clamp the metering guide sleeve 60. Whereby the driving spiral tube 30, the clutch sleeve 40, and the metering guide sleeve 60 can be rotated forwardly together. Wherein, because of the threading connection between the driving spiral guiding groove 33 at an external peripheral surface of the driving spiral tube 30 and the spiral guiding rib 25 at an inner peripheral surface of the housing 20, when the driving spiral tube 30 spirally moves forward inside the housing 20, the number of revolutions of the driving spiral tube 30 is equal to the number of revolutions of the total volume control nut 70, around the metering guide sleeve 60, driven by the clutch sleeve 40. The metering guide sleeve 60 is pushed by the diagonal tension knob 80, so the non-return ratchet buckles 62 of the metering guide sleeve 60 expand to engage with the one-way ratchet portion 23 of the housing 20. The metering guide sleeve 60 can only rotate at the fixing point inside the housing 20 and drive the metering screw 90 to linearly move forward to push the piston in the medicine vial 3 to output a predetermined dose of the medicament liquid.

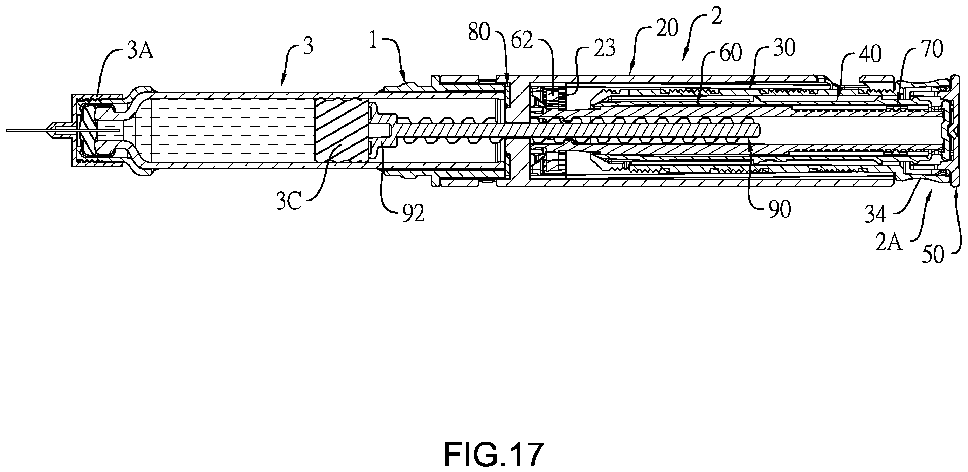

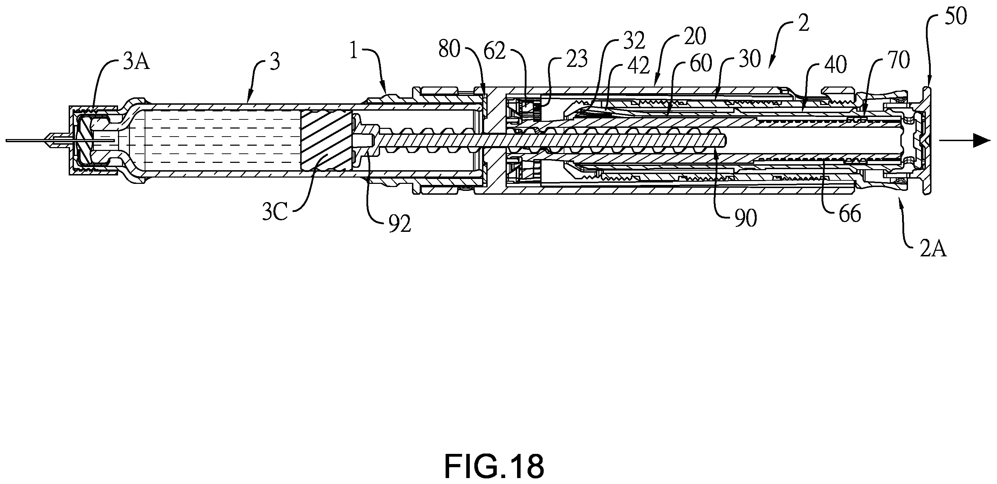

As shown in Figs. 17, 18 and 6 to 8, after the output of the predetermined dose of the medicament liquid is completed once, the user releases the output button 50 at the rear end of the spiral pushing assembly 2A. The flexible locking claws 42 at the front end of the clutch sleeve 40 are guided by the guiding curved surface 32 at the front end of the driving spiral tube 30, the clutch sleeve 40 moves backwardly and the flexible locking claws 42 expand to release the metering guide sleeve 60. Accordingly, the spiral pushing assembly 2A is able to be pulled backwardly to perform next injection of predetermined dose of the medicament liquid.

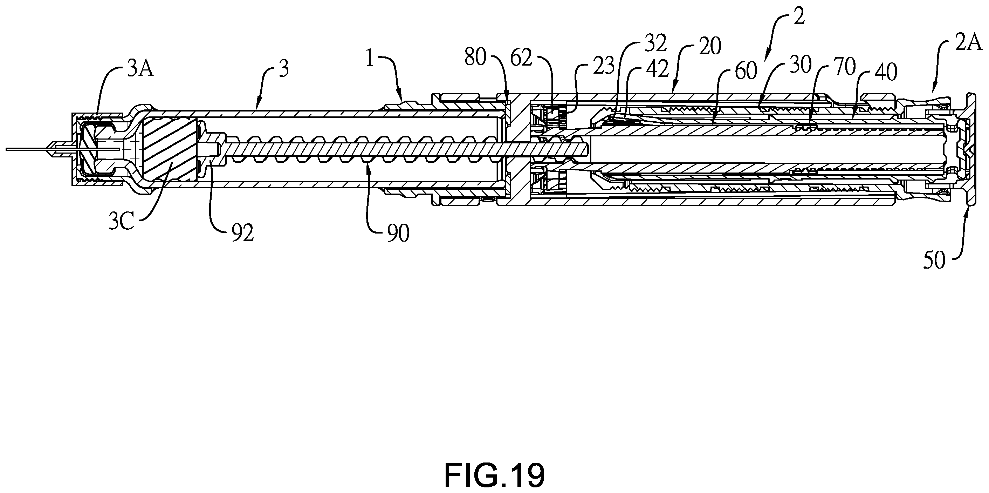

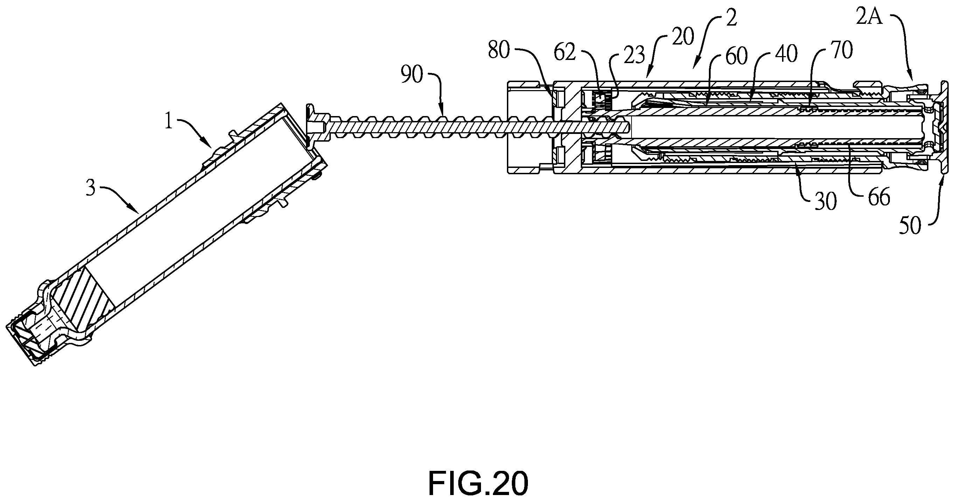

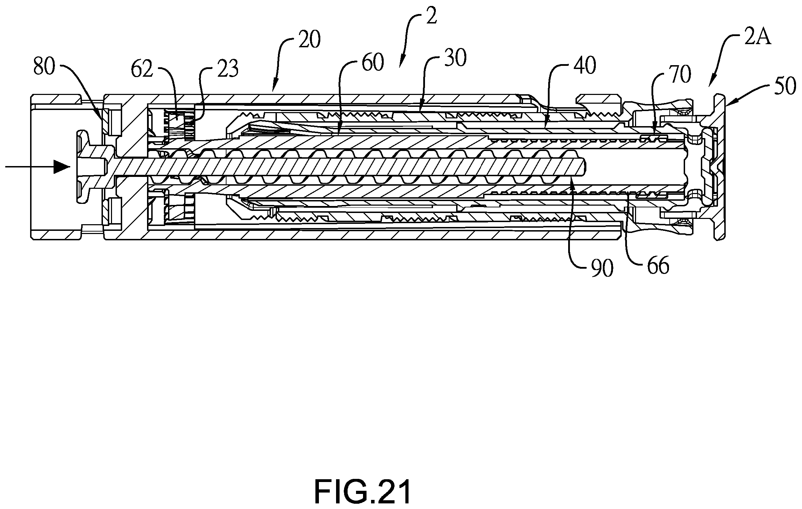

As shown in Figs. 19 to 21 and 6 to 8, after used for multiple times, the medicament liquid in the medicine vial 3 is used up, and the user detaches the cartridge sleeve 1 and the medicine vial 3 therein from the front end of the housing 20. At this time, the force from the cartridge sleeve 1 and subjected to the diagonal tension knob 80 is removed. The diagonal tension knob 80 is subjected to a restoring elastic force from the contraction of the non-return ratchet buckles 62 of the metering guide sleeve 60 and is pushed forward to a default position. Because the non-return ratchet buckles 62 disengage from the one-way ratchet portion 23 of the housing 20, the unidirectional restriction of the metering guide sleeve 60 is released to convert into the standby mode. At this time, the axial guiding groove 94 is aligned with the guiding block 221 in the guiding ring 22 of the housing 20. The metering screw 90 extending out from the housing 20 is pushed back to the default position along the central axis. The metering guide sleeve 60 rotates backwardly to push the total volume control nut 70 back to an initial full position of the metering guide sleeve 60 along the central axis A. Accordingly, the syringe can be reused.

Anyone familiar with the professional technology, without departing from the scope of the technical solution of the present invention, can make use of the technical content disclosed above to make slight changes or modification into equivalent embodiments with equivalent changes, but any content that does not depart from the technical solution of the present invention is based on the present invention. Any simple modifications, equivalent changes and modifications made to the above embodiments by technical essence still fall within the scope of the technical solutions of the present invention.

## Claims

1. A syringe detachably assembled with a medicine vial (3) containing medicament liquid and a needle component (3A) connected to a front end o1f the medicine vial (3), wherein the syringe defines a central axis (A) extending along a front-to-back direction of the syringe; the syringe comprises an injection mechanism (2); the injection mechanism (2) has an injection mode and a standby mode, and is able to be manually triggered to perform a conversion between the injection mode and the standby mode; the injection mechanism (2) comprises:
a housing (20) having a movable space (200) formed through the housing (20) along the central axis (A), a front end of the housing (20) detachably connected to the medicine vial (3); and a one-way ratchet portion (23) formed on a peripheral wall of the movable space (200) at a front segment of the movable space (200) of the housing (20), and the housing (20) having at least one spiral guiding rib (25) formed on an inner peripheral wall of the housing (20);
a spiral pushing assembly (2A) mounted in the movable space (200) of the housing (20) and being capable of spirally moving around the central axis (A); the spiral pushing assembly (2A) extending to an outside of a rear end of the housing (20); and the spiral pushing assembly (2A) being able to be pushed to generate a spiral propulsion, the spiral pushing assembly (2A) comprising a driving spiral tube (30); the driving spiral tube (30) mounted in and being capable of spirally moving in the movable space (200) of the housing (20); the driving spiral tube (30) having a driving spiral guiding groove (33) formed in an external peripheral wall of the driving spiral tube (30) and screwed with the at least one spiral guiding rib (25) of the housing (20);
a metering guide sleeve (60) mounted in the housing (20), being capable of rotating at a fixing point; and the metering guide sleeve (60) inserted into the spiral pushing assembly (2A) and able to be controlled to connect and rotate with the spiral pushing assembly (2A); the metering guide sleeve (60) comprising a guide sleeve body (61) and multiple non-return ratchet buckles (62); the guide sleeve body (61) having a guide sleeve central hole (63), at least one spiral portion (65) formed on an inner peripheral wall of the guide sleeve central hole (63); a guide sleeve threaded portion (66) formed on a rear segment of an outer peripheral surface of the guide sleeve body (61); and a guide sleeve front end portion (64) formed at a front end of the guide sleeve body (61); the multiple non-return ratchet buckles (62) formed on an external surface of the guide sleeve front end portion (64); when the multiple non-return ratchet buckles (62) controlled to engage with the one-way ratchet portion (23) of the housing (20), the metering guide sleeve (60) solely driven to rotate forwardly and in said injection mode; when the multiple non-return ratchet buckles (62) disengaging from the one-way ratchet portion (23) of the housing (20), the metering guide sleeve (60) able to rotate forwardly or backwardly and in said standby mode;
a total volume control nut (70) mounted in the spiral pushing assembly (2A), being capable of linearly moving along the central axis (A), and screwed with the guide sleeve threaded portion (66) at a rear segment of the metering guide sleeve (60);
a diagonal tension knob (80) mounted in the front end of the housing (20) and being movable along the central axis (A); the diagonal tension knob (80) being drivable to change an engaging condition of the non-return ratchet buckles (62) relative to the one-way ratchet portion (23) of the housing (20) and to convert the said injection mode and the said standby mode; and
a metering screw (90) inserted in the diagonal tension knob (80) and the housing (20) and the metering screw (90) screwed within the metering guide sleeve (60); the metering screw (90) having a screw body (91) having a screw threaded portion (93) and the metering screw (90) screwed with said spiral portion (65) inside the metering guide sleeve (60) via the screw threaded portion (93); a front end of the metering screw (90) extending in front of the diagonal tension knob (80); when the injection mechanism (2) in the injection mode, the diagonal tension knob (80) being triggered to move backward and to drive the multiple non-return ratchet buckles (62) to engage with the one-way ratchet portion (23) of the housing (20), a spiral movement of the metering guide sleeve (60) being able to drive the metering screw (90) to move linearly along the central axis (A) inside the housing (20) to perform a quantitative output of the medicament liquid in the medicine vial (3); when the injection mechanism (2) in the standby mode, the diagonal tension knob (80) being able to move forward to make the multiple non-return ratchet buckles (62) disengage from the one-way ratchet portion (23) of the housing (20), the metering screw (90) being able to linearly move backward and be received into the housing (20) along the central axis (A).

2. The syringe as claimed in claim 1, **characterized in that**, the syringe comprises a cartridge sleeve (1); the cartridge sleeve (1) has a containing space (100) formed through and within the cartridge sleeve (1) along the central axis (A); the cartridge sleeve (1) is detachably installed to the medicine vial (3), and a front end of the cartridge sleeve (1) is detachably installed to the needle component (3A); the cartridge sleeve (1) is able to be connected to the front end of the housing (20) of the injection mechanism (2); and the cartridge sleeve (1) is able to trigger the diagonal tension knob (80) to move backward and to drive the multiple non-return ratchet buckles (62) to engage with the one-way ratchet portion (23) of the housing (20) and in the said injection mode; and when the cartridge sleeve (1) is detached from the front end of the housing (20) of the injection mechanism (2), the diagonal tension knob (80) is able to move forward to make the multiple non-return ratchet buckles (62) disengage from the one-way ratchet portion (23) of the housing (20) and in the said standby mode.

3. The syringe as claimed in claim 2, **characterized in that**, the cartridge sleeve (1) comprises a cartridge body (10); the cartridge body (10) has at least one viewing window (14) formed in a peripheral wall of the cartridge body (10); a connection portion (12) is formed at a rear end of the cartridge body (10); a positioning flange (13) is formed on the peripheral wall of the cartridge body (10) and located at a front end of the connection portion (12); multiple engaging protrusions (15) are formed on an outer peripheral surface of the connection portion (12); the front end of the housing (20) is a cartridge sleeve connecting portion (21); multiple L-shaped engaging grooves (211) are formed in an inner peripheral wall of the cartridge sleeve connecting portion (21), each L-shaped engaging groove (211) includes a straight groove section being parallel to the central axis (A) and a transverse section connected to a rear end of the straight groove section; when the connection portion (12) at the rear end of the cartridge sleeve (1) is inserted into the cartridge sleeve connecting portion (21) at the front end of the housing (20), each engaging protrusion (15) is able to slide along the straight groove section of a respective one of the L-shaped engaging grooves (211) and to turn into an end of the transverse section of the respective one of the L-shaped engaging grooves (211) to lock the cartridge sleeve (1) to the front end of the housing (20).

4. The syringe as claimed in claim 1, **characterized in that**, a guiding ring (22) is formed in the front end of the housing (20); an outer peripheral surface of the guiding ring (22) is connected to the peripheral wall of the movable space (200) via multiple arms (24); a spacing is formed between the guiding ring (22) and the peripheral wall of the movable space (200); at least one guiding block (221) is formed on an inner annular wall of the guiding ring (22); the screw body (91) of the metering screw (90) has at least one axial guiding groove (94) passing through the screw threaded portion (93) and being parallel to the central axis (A); said axial guiding groove (94) engages with said guiding block (221) inside the guiding ring (22) of the housing (20).

5. The syringe as claimed in claim 4, **characterized in that**, each non-return ratchet buckle (62) has a wing (621) protruding forwardly and disposed at a front end face of the non-return ratchet buckle (62); the diagonal tension knob (80) has a conical surface (82) abutting against the wing (621) of each non-return ratchet buckle (62) to let the conical surface (82) of the diagonal tension knob (80) push the non-return ratchet buckle (62) to engage with the one-way ratchet portion (23) of the housing (20) via the wing (621).

6. The syringe as claimed in claim 5, **characterized in that**, the syringe comprises a cartridge sleeve (1); the cartridge sleeve (1) has a containing space (100) formed through and within the cartridge sleeve (1) along the central axis (A); the cartridge sleeve (1) is detachably installed to the medicine vial (3), and a front end of the cartridge sleeve (1) is detachably installed to the needle component (3A); the cartridge sleeve (1) is able to be connected to the front end of the housing (20) of the injection mechanism (2); and the cartridge sleeve (1) is able to trigger the diagonal tension knob (80) to move backward and to drive the multiple non-return ratchet buckles (62) to engage with the one-way ratchet portion (23) of the housing (20) and in the said injection mode; and when the cartridge sleeve (1) is detached from the front end of the housing (20) of the injection mechanism (2), the diagonal tension knob (80) is able to move forward to make the multiple non-return ratchet buckles (62) disengage from the one-way ratchet portion (23) of the housing (20) and in the said standby mode.

7. The syringe as claimed in claim 6, **characterized in that**,
the cartridge sleeve (1) comprises a cartridge body (10); the cartridge body (10) has at least one viewing window (14) formed in a peripheral wall of the cartridge body (10); a connection portion (12) is formed at a rear end of the cartridge body (10); a positioning flange (13) is formed on the peripheral wall of the cartridge body (10) and located at a front end of the connection portion (12); multiple engaging protrusions (15) are formed on an outer peripheral surface of the connection portion (12); the front end of the housing (20) is a cartridge sleeve connecting portion (21); multiple L-shaped engaging grooves (211) are formed in an inner peripheral wall of the cartridge sleeve connecting portion (21), each L-shaped engaging groove (211) includes a straight groove section being parallel to the central axis (A) and a transverse section connected to a rear end of the straight groove section; when the connection portion (12) at the rear end of the cartridge sleeve (1) is inserted into the cartridge sleeve connecting portion (21) at the front end of the housing (20), each engaging protrusion (15) is able to slide along the straight groove section of a respective one of the L-shaped engaging grooves (211) and to turn into an end of the transverse section of the respective one of the L-shaped engaging grooves (211) to lock the cartridge sleeve (1) to the front end of the housing (20).

8. The syringe as claimed in any one of claims 1 to 7, **characterized in that** the spiral pushing assembly (2A) comprises a clutch sleeve (40), and an output button (50), wherein:
the driving spiral tube (30) has a spiral tube channel (31) formed through the driving spiral tube (30) along the central axis (A), a guiding curved surface (32) formed at a front segment of the spiral tube channel (31) and converging forwardly along the central axis (A), and a spiral tube rear end portion (34) disposed at a rear end of the driving spiral tube (30); the spiral tube rear end portion (34) extends to an outside of the rear end of the housing (20) and is blocked by the rear end of the housing (20);
the clutch sleeve (40) is mounted in the spiral tube channel (31) of the driving spiral tube (30) and is able to move linearly relative to the driving spiral tube (30); the clutch sleeve (40) has a sleeve body (41), multiple flexible locking claws (42) formed at a front end of the sleeve body (41), and a connecting end portion (43) disposed at a rear end of the sleeve body (41); a sleeve channel (44) is formed in the sleeve body (41) and extends along the central axis (A); at least one sleeve guide groove portion (45) is formed on a peripheral wall of the sleeve channel (44) and is parallel to the central axis (A); the multiple flexible locking claws (42) have flexibility for radial expansion and contraction; when the clutch sleeve (40) is moved frontward relative to the driving spiral tube (30), the multiple flexible locking claws (42) are abutted by the guiding curved surface (32) at a front end of the driving spiral tube (30) and contract radially; and the multiple flexible locking claws (42) are capable of bouncing backward to an original position along the guiding curved surface (32) and expand;
the output button (50) is disposed at an outside behind the rear end of the housing (20) and is connected with the connecting end portion (43) at a rear end of the clutch sleeve (40);
the metering guide sleeve (60) is inserted in the driving spiral tube (30) and the clutch sleeve (40) and is able to be clamped by the multiple flexible locking claws (42) of the clutch sleeve (40);
the total volume control nut (70) has at least one flange rib (71) formed on an outer peripheral surface of the total volume control nut (70) and engaging with said at least one sleeve guide groove portion (45) of the clutch sleeve (40).

9. The syringe as claimed in claim 8, **characterized in that**, each of the flexible locking claws (42) has a locking surface (421) formed at an inner surface of the flexible locking claw (42); the locking surface (421) of each of the flexible locking claws (42) has multiple straight stripes extending along a front-to-back direction; the metering guide sleeve (60) has an engaging surface (67) formed on the outer peripheral surface, located between the guide sleeve front end portion (64) and the guide sleeve threaded portion (66), of the guide sleeve body (61); the engaging surface (67) has multiple straight stripes being parallel to the central axis (A) and corresponding to the straight stripes of the locking surfaces (421) of the flexible locking claws (42) for engagement.

10. The syringe as claimed in claim 8, **characterized in that**, the housing (20) has a scale observing window (26) formed at a rear section of an external peripheral wall of the housing (20); the driving spiral tube (30) has a spiral scale portion (35) formed on the external peripheral wall of the driving spiral tube (30) and being parallel to the driving spiral guiding groove (33); the spiral scale portion (35) has multiple scale lines able to be observed through the scale observing window (26) of the housing (20).

## Patentansprüche

1. Spritze, die lösbar mit einer medizinischen Durchstechflasche (3), die eine Medikamentenflüssigkeit enthält, und einer Nadelkomponente (3A), die mit einem vorderen Ende der medizinischen Durchstechflasche (3) verbunden ist, montiert ist, wobei
die Spritze eine Mittelachse (A) definiert, die sich entlang einer von vorne nach hinten verlaufenden Richtung der Spritze erstreckt; die Spritze einen Injektionsmechanismus (2) umfasst; der Injektionsmechanismus (2) einen Injektionsmodus und einen Standby-Modus aufweist und manuell ausgelöst werden kann, um einen Wechsel zwischen dem Injektionsmodus und dem Standby-Modus durchzuführen; wobei der Injektionsmechanismus (2) umfasst:
ein Gehäuse (20), das einen beweglichen Raum (200) aufweist, der entlang der Mittelachse (A) durch das Gehäuse (20) gebildet ist, wobei ein vorderes Ende des Gehäuses (20) lösbar mit der medizinischen Durchstechflasche (3) verbunden ist; und einen Einweg-Sperrradabschnitt (23), der an einer Umfangswand des beweglichen Raums (200) an einem vorderen Segment des beweglichen Raums (200) des Gehäuses (20) gebildet ist, und wobei das Gehäuse (20) mindestens eine spiralförmige Führungsrippe (25) aufweist, die an einer inneren Umfangswand des Gehäuses (20) gebildet ist;
eine spiralförmige Schubanordnung (2A), die in dem beweglichen Raum (200) des Gehäuses (20) angebracht ist und sich spiralförmig um die Mittelachse (A) bewegen kann; wobei sich die spiralförmige Schubanordnung (2A) zu einer Außenseite eines hinteren Endes des Gehäuses (20) erstreckt; und wobei die spiralförmige Schubanordnung (2A) gedrückt werden kann, um einen spiralförmigen Antrieb zu erzeugen, wobei die spiralförmige Schubanordnung (2A) ein spiralförmiges Antriebsröhrchen (30) umfasst; wobei das spiralförmige Antriebsröhrchen (30) in dem beweglichen Raum (200) des Gehäuses (20) angebracht ist und sich spiralförmig darin bewegen kann; wobei das spiralförmige Antriebsröhrchen (30) eine spiralförmige Antriebsführungsnut (33) aufweist, die in einer äußeren Umfangswand des spiralförmigen Antriebsröhrchens (30) gebildet ist und mit der mindestens einen spiralförmigen Führungsrippe (25) des Gehäuses (20) verschraubt ist;
eine Dosierführungshülse (60), die in dem Gehäuse (20) angebracht ist und an einem Befestigungspunkt drehbar ist; und wobei die Dosierführungshülse (60) in die spiralförmige Schubanordnung (2A) eingeführt ist und so gesteuert werden kann, dass sie mit der spiralförmigen Schubanordnung (2A) verbunden ist und sich mit dieser dreht; wobei die Dosierführungshülse (60) einen Führungshülsenkörper (61) und mehrere Rücklaufsperrklinken (62) umfasst;
wobei der Führungshülsenkörper (61) ein Führungshülsenmittelloch (63) aufweist, wobei mindestens ein spiralförmiger Abschnitt (65) an einer inneren Umfangswand des Führungshülsenmittellochs (63) gebildet ist; einen Führungshülsengewindeabschnitt (66), der an einem hinteren Segment einer äußeren Umfangsfläche des Führungshülsenkörpers (61) gebildet ist; und einen vorderen Führungshülsenendabschnitt (64), der an einem vorderen Ende des Führungshülsenkörpers (61) gebildet ist; wobei die mehreren Rücklaufsperrklinken (62) an einer Außenfläche des vorderen Führungshülsenendabschnitts (64) gebildet sind; wobei, wenn die mehreren Rücklaufsperrklinken (62) so gesteuert werden, dass sie mit dem Einweg-Sperrradabschnitt (23) des Gehäuses (20) in Eingriff stehen, die Dosierführungshülse (60) ausschließlich angetrieben wird, um sich vorwärts und in dem Injektionsmodus zu drehen; wobei, wenn die mehreren Rücklaufsperrklinken (62) von dem Einweg-Sperrradabschnitt (23) des Gehäuses (20) ausgerückt sind, sich die Dosierführungshülse (60) vorwärts oder rückwärts und in dem Standby-Modus drehen kann;
eine Gesamtvolumensteuerungsmutter (70), die in der spiralförmigen Schubanordnung (2A) angebracht ist, sich linear entlang der Mittelachse (A) bewegen kann und mit dem Führungshülsengewindeabschnitt (66) an einem hinteren Segment der Dosierführungshülse (60) verschraubt ist;
einen Diagonalspannknopf (80), der in dem vorderen Ende des Gehäuses (20) angebracht ist und entlang der Mittelachse (A) bewegbar ist; wobei der Diagonalspannknopf (80) betätigbar ist, um einen Eingriffszustand der Rücklaufsperrklinken (62) relativ zu dem Einweg-Sperrradabschnitt (23) des Gehäuses (20) zu ändern und den Injektionsmodus und den Standby-Modus zu wechseln; und
eine Dosierschnecke (90), die in den Diagonalspannknopf (80) und das Gehäuse (20) eingeführt ist, und wobei die Dosierschnecke (90) in die Dosierführungshülse (60) eingeschraubt ist; wobei die Dosierschnecke (90) einen Schneckenkörper (91) mit einem Schneckengewindeabschnitt (93) aufweist und die Dosierschnecke (90) über den Schneckengewindeabschnitt (93) mit dem spiralförmigen Abschnitt (65) innerhalb der Dosierführungshülse (60) verschraubt ist; wobei sich ein vorderes Ende der Dosierschnecke (90) vor dem Diagonalspannknopf (80) erstreckt; wobei, wenn sich der Injektionsmechanismus (2) im Injektionsmodus befindet, der Diagonalspannknopf (80) ausgelöst wird, um sich nach hinten zu bewegen und die mehreren Rücklaufsperrklinken (62) anzutreiben, um sie mit dem Einweg-Sperrradabschnitt (23) des Gehäuses (20) in Eingriff zu bringen, wobei eine spiralförmige Bewegung der Dosierführungshülse (60) die Dosierschnecke (90) dazu antreiben kann, sich linear entlang der Mittelachse (A) im Inneren des Gehäuses (20) zu bewegen, um eine quantitative Abgabe der Medikamentenflüssigkeit in der medizinischen Durchstechflasche (3) durchzuführen; wobei, wenn sich der Injektionsmechanismus (2) im Standby-Modus befindet, sich der Diagonalspannknopf (80) nach vorne bewegen kann, um die mehreren Rücklaufsperrklinken (62) aus dem Einweg-Sperrradabschnitt (23) des Gehäuses (20) auszurücken, wobei sich die Dosierschnecke (90) linear nach hinten bewegen und entlang der Mittelachse (A) in das Gehäuse (20) aufgenommen werden kann.

2. Spritze nach Anspruch 1, **dadurch gekennzeichnet, dass** die Spritze eine Kartuschenhülse (1) umfasst; die Kartuschenhülse (1) einen Aufnahmeraum (100) aufweist, der entlang der Mittelachse (A) durch die Kartuschenhülse (1) hindurch und innerhalb dieser gebildet ist; die Kartuschenhülse (1) lösbar an der medizinischen Durchstechflasche (3) installiert ist und ein vorderes Ende der Kartuschenhülse (1) lösbar an der Nadelkomponente (3A) installiert ist; die Kartuschenhülse (1) mit dem vorderen Ende des Gehäuses (20) des Injektionsmechanismus (2) verbunden werden kann; und die Kartuschenhülse (1) den Diagonalspannknopf (80) auslösen kann, um sich nach hinten zu bewegen und die mehreren Rücklaufsperrklinken (62) anzutreiben, um mit dem Einweg-Sperrradabschnitt (23) des Gehäuses (20) in Eingriff und in den Injektionsmodus zu kommen; und wobei, wenn die Kartuschenhülse (1) von dem vorderen Ende des Gehäuses (20) des Injektionsmechanismus (2) gelöst wird, sich der Diagonalspannknopf (80) nach vorne bewegen kann, um die mehreren Rücklaufsperrklinken (62) aus dem Einweg-Sperrradabschnitt (23) des Gehäuses (20) auszurücken und in den Standby-Modus zu bringen.

3. Spritze nach Anspruch 2, **dadurch gekennzeichnet, dass** die Kartuschenhülse (1) einen Kartuschenkörper (10) umfasst; der Kartuschenkörper (10) mindestens ein Sichtfenster (14) aufweist, das in einer Umfangswand des Kartuschenkörpers (10) gebildet ist; ein Verbindungsabschnitt (12) an einem hinteren Ende des Kartuschenkörpers (10) gebildet ist; ein Positionierungsflansch (13) an der Umfangswand des Kartuschenkörpers (10) gebildet ist und sich an einem vorderen Ende des Verbindungsabschnitts (12) befindet; an einer äußeren Umfangsfläche des Verbindungsabschnitts (12) mehrere Eingriffsvorsprünge (15) gebildet sind; das vordere Ende des Gehäuses (20) ein Kartuschenhülsenverbindungsabschnitt (21) ist; mehrere L-förmige Eingriffsnuten (211) in einer inneren Umfangswand des Kartuschenhülsenverbindungsabschnitts (21) gebildet sind, wobei jede L-förmige Eingriffsnut (211) einen geraden Nutteilabschnitt, der parallel zu der Mittelachse (A) verläuft, und einen Querteilabschnitt umfasst, der mit einem hinteren Ende des geraden Nutteilabschnitts verbunden ist; wobei, wenn der Verbindungsabschnitt (12) am hinteren Ende der Kartuschenhülse (1) in den Kartuschenhülsenverbindungsabschnitt (21) am vorderen Ende des Gehäuses (20) eingeführt ist, jeder Eingriffsvorsprung (15) entlang des geraden Nutteilabschnitts einer jeweiligen der L-förmigen Eingriffsnuten (211) gleiten kann und in ein Ende des Querteilabschnitts einer jeweiligen der L-förmigen Eingriffsnuten (211) einrasten kann, um die Kartuschenhülse (1) an dem vorderen Ende des Gehäuses (20) zu verriegeln.

4. Spritze nach Anspruch 1, **dadurch gekennzeichnet, dass** ein Führungsring (22) in dem vorderen Ende des Gehäuses (20) gebildet ist; eine äußere Umfangsfläche des Führungsrings (22) über mehrere Arme (24) mit der Umfangswand des beweglichen Raums (200) verbunden ist; ein Abstand zwischen dem Führungsring (22) und der Umfangswand des beweglichen Raums (200) gebildet ist; mindestens ein Führungsblock (221) an einer inneren Ringwand des Führungsrings (22) gebildet ist; der Schneckenkörper (91) der Dosierschnecke (90) mindestens eine axiale Führungsnut (94) aufweist, die durch den Schneckengewindeabschnitt (93) verläuft und parallel zu der Mittelachse (A) ist; wobei die axiale Führungsnut (94) mit dem Führungsblock (221) innerhalb des Führungsrings (22) des Gehäuses (20) in Eingriff steht.

5. Spritze nach Anspruch 4, **dadurch gekennzeichnet, dass** jede Rücklaufsperrklinke (62) einen nach vorne hervorstehenden Flügel (621) aufweist, der an einer vorderen Stirnfläche der Rücklaufsperrklinke (62) angeordnet ist; der Diagonalspannknopf (80) eine konische Fläche (82) aufweist, die an dem Flügel (621) jeder Rücklaufsperrklinke (62) anliegt, damit die konische Fläche (82) des Diagonalspannknopfs (80) die Rücklaufsperrklinke (62) über den Flügel (621) in Eingriff mit dem Einweg-Sperrradabschnitt (23) des Gehäuses (20) drückt.

6. Spritze nach Anspruch 5, **dadurch gekennzeichnet, dass** die Spritze eine Kartuschenhülse (1) umfasst; die Kartuschenhülse (1) einen Aufnahmeraum (100) aufweist, der entlang der Mittelachse (A) durch die Kartuschenhülse (1) hindurch und innerhalb dieser gebildet ist; die Kartuschenhülse (1) lösbar an der medizinischen Durchstechflasche (3) installiert ist und ein vorderes Ende der Kartuschenhülse (1) lösbar an der Nadelkomponente (3A) installiert ist; die Kartuschenhülse (1) mit dem vorderen Ende des Gehäuses (20) des Injektionsmechanismus (2) verbunden werden kann; und die Kartuschenhülse (1) den Diagonalspannknopf (80) auslösen kann, um sich nach hinten zu bewegen und die mehreren Rücklaufsperrklinken (62) anzutreiben, um mit dem Einweg-Sperrradabschnitt (23) des Gehäuses (20) in Eingriff und in den Injektionsmodus zu kommen; und wobei, wenn die Kartuschenhülse (1) von dem vorderen Ende des Gehäuses (20) des Injektionsmechanismus (2) gelöst wird, sich der Diagonalspannknopf (80) nach vorne bewegen kann, um die mehreren Rücklaufsperrklinken (62) aus dem Einweg-Sperrradabschnitt (23) des Gehäuses (20) auszurücken und in den Standby-Modus zu bringen.

7. Spritze nach Anspruch 6, **dadurch gekennzeichnet, dass** die Kartuschenhülse (1) einen Kartuschenkörper (10) umfasst; der Kartuschenkörper (10) mindestens ein Sichtfenster (14) aufweist, das in einer Umfangswand des Kartuschenkörpers (10) gebildet ist; ein Verbindungsabschnitt (12) an einem hinteren Ende des Kartuschenkörpers (10) gebildet ist; ein Positionierungsflansch (13) an der Umfangswand des Kartuschenkörpers (10) gebildet ist und sich an einem vorderen Ende des Verbindungsabschnitts (12) befindet; an einer äußeren Umfangsfläche des Verbindungsabschnitts (12) mehrere Eingriffsvorsprünge (15) gebildet sind; das vordere Ende des Gehäuses (20) ein Kartuschenhülsenverbindungsabschnitt (21) ist; mehrere L-förmige Eingriffsnuten (211) in einer inneren Umfangswand des Kartuschenhülsenverbindungsabschnitts (21) gebildet sind, wobei jede L-förmige Eingriffsnut (211) einen geraden Nutteilabschnitt, der parallel zu der Mittelachse (A) verläuft, und einen Querteilabschnitt umfasst, der mit einem hinteren Ende des geraden Nutteilabschnitts verbunden ist; wobei, wenn der Verbindungsabschnitt (12) am hinteren Ende der Kartuschenhülse (1) in den Kartuschenhülsenverbindungsabschnitt (21) am vorderen Ende des Gehäuses (20) eingeführt ist, jeder Eingriffsvorsprung (15) entlang des geraden Nutteilabschnitts einer jeweiligen der L-förmigen Eingriffsnuten (211) gleiten kann und in ein Ende des Querteilabschnitts einer der jeweiligen L-förmigen Eingriffsnuten (211) einrasten kann, um die Kartuschenhülse (1) an dem vorderen Ende des Gehäuses (20) zu verriegeln.

8. Spritze nach einem der Ansprüche 1 bis 7, **dadurch gekennzeichnet, dass** die spiralförmige Schubanordnung (2A) eine Kupplungshülse (40) und einen Abgabeknopf (50) umfasst, wobei:
das spiralförmige Antriebsröhrchen (30) einen spiralförmigen Röhrchenkanal (31), der entlang der Mittelachse (A) durch das spiralförmige Antriebsröhrchen (30) hindurch gebildet ist, eine gekrümmte Führungsfläche (32), die an einem vorderen Segment des spiralförmigen Röhrchenkanals (31) gebildet ist und entlang der Mittelachse (A) nach vorne konvergiert, und einen hinteren Endabschnitt (34) des spiralförmigen Röhrchens aufweist, der an einem hinteren Ende des spiralförmigen Antriebsröhrchens (30) angeordnet ist; sich der hintere Endabschnitt (34) des spiralförmigen Röhrchens bis zu einer Außenseite des hinteren Endes des Gehäuses (20) erstreckt und durch das hintere Ende des Gehäuses (20) blockiert wird;
die Kupplungshülse (40) in dem spiralförmigen Röhrchenkanal (31) des spiralförmigen Antriebsröhrchens (30) angebracht ist und sich relativ zu dem spiralförmigen Antriebsröhrchen (30) linear bewegen kann; die Kupplungshülse (40) einen Hülsenkörper (41), mehrere flexible Verriegelungsklauen (42), die an einem vorderen Ende des Hülsenkörpers (41) gebildet sind, und einen Verbindungsendabschnitt (43) aufweist, der an einem hinteren Ende des Hülsenkörpers (41) angeordnet ist; ein Hülsenkanal (44) im Hülsenkörper (41) gebildet ist und sich entlang der Mittelachse (A) erstreckt; mindestens ein Hülsenführungsnutabschnitt (45) an einer Umfangswand des Hülsenkanals (44) gebildet ist und parallel zu der Mittelachse (A) verläuft; die mehreren flexiblen Verriegelungsklauen (42) eine Flexibilität zur radialen Ausdehnung und Kontraktion aufweisen; wobei, wenn die Kupplungshülse (40) relativ zu dem spiralförmigen Antriebsröhrchen (30) nach vorne bewegt wird, die mehreren flexiblen Verriegelungsklauen (42) an der gekrümmten Führungsfläche (32) an dem vorderen Ende des spiralförmigen Antriebsröhrchens (30) anliegen und sich radial zusammenziehen; und die mehreren flexiblen Verriegelungsklauen (42) entlang der gekrümmten Führungsfläche (32) nach hinten in eine Ausgangsposition zurückspringen und sich ausdehnen können;
der Abgabeknopf (50) an einer Außenseite hinter dem hinteren Ende des Gehäuses (20) angeordnet ist und mit dem Verbindungsendabschnitt (43) an einem hinteren Ende der Kupplungshülse (40) verbunden ist;
die Dosierführungshülse (60) in das spiralförmige Antriebsröhrchen (30) und die Kupplungshülse (40) eingeführt ist und durch die mehreren flexiblen Verriegelungsklauen (42) der Kupplungshülse (40) festgeklemmt werden kann;
die Gesamtvolumensteuerungsmutter (70) mindestens eine Flanschrippe (71) aufweist, die an einer äußeren Umfangsfläche der Gesamtvolumensteuerungsmutter (70) gebildet ist und mit dem mindestens einen Hülsenführungsnutabschnitt (45) der Kupplungshülse (40) in Eingriff steht.

9. Spritze nach Anspruch 8, **dadurch gekennzeichnet, dass** jede der flexiblen Verriegelungsklauen (42) eine Verriegelungsfläche (421) aufweist, die an einer Innenfläche der flexiblen Verriegelungsklaue (42) gebildet ist;
die Verriegelungsfläche (421) jeder der flexiblen Verriegelungsklauen (42) mehrere gerade Streifen aufweist, die sich in einer von vorne nach hinten verlaufenden Richtung erstrecken; die Dosierführungshülse (60) eine Eingriffsfläche (67) aufweist, die an der äußeren Umfangsfläche des Führungshülsenkörpers (61) gebildet ist, die sich zwischen dem vorderen Führungshülsenendabschnitt (64) und dem Führungshülsengewindeabschnitt (66) befindet; die Eingriffsfläche (67) mehrere gerade Streifen aufweist, die parallel zu der Mittelachse (A) verlaufen und den geraden Streifen der Verriegelungsflächen (421) der flexiblen Verriegelungsklauen (42) zum Eingriff entsprechen.

10. Spritze nach Anspruch 8, **dadurch gekennzeichnet, dass** das Gehäuse (20) ein Skalenbeobachtungsfenster (26) aufweist, das an einem hinteren Teilabschnitt einer äußeren Umfangswand des Gehäuses (20) gebildet ist; das spiralförmige Antriebsröhrchen (30) einen spiralförmigen Skalenabschnitt (35) aufweist, der an der äußeren Umfangswand des spiralförmigen Antriebsröhrchens (30) gebildet ist und parallel zu der spiralförmigen Antriebsführungsnut (33) verläuft; wobei der spiralförmige Skalenabschnitt (35) mehrere Skalenlinien aufweist, die durch das Skalenbeobachtungsfenster (26) des Gehäuses (20) beobachtet werden können.

## Revendications

1. Seringue assemblée de manière amovible avec un flacon de médicament (3) contenant un médicament liquide et un composant d'aiguille (3A) relié à une extrémité avant du flacon de médicament (3), dans laquelle
la seringue définit un axe central (A) s'étendant le long d'une direction avant-arrière de la seringue : la seringue comprend un mécanisme d'injection (2) ; le mécanisme d'injection (2) a un mode injection et un mode veille, et peut être déclenché manuellement pour effectuer une conversion entre le mode injection et le mode veille ; le mécanisme d'injection (2) comprend :
un boîtier (20) ayant un espace mobile (200) formé à travers le boîtier (20) le long de l'axe central (A), une extrémité avant du boîtier (20) reliée de manière amovible au flacon de médicament (3) ; et une portion à cliquet à sens unique (23) formée sur une paroi périphérique de l'espace mobile (200) au niveau d'un segment avant de l'espace mobile (200) du boîtier (20), et le boîtier (20) ayant au moins une nervure de guidage en spirale (25) formée sur une paroi périphérique interne du boîtier (20) ;
un ensemble de poussée en spirale (2A) monté dans l'espace mobile (200) du boîtier (20) et pouvant se déplacer en spirale autour de l'axe central (A) ;
l'ensemble de poussée en spirale (2A) s'étendant vers un extérieur d'une extrémité arrière du boîtier (20) ; et l'ensemble de poussée en spirale (2A) pouvant être poussé pour générer une propulsion en spirale, l'ensemble de poussée en spirale (2A) comprenant un tube en spirale d'entraînement (30) ; le tube en spirale d'entraînement (30) monté dans et pouvant se déplacer en spirale dans l'espace mobile (200) du boîtier (20) ; le tube en spirale d'entraînement (30) ayant une rainure de guidage en spirale d'entraînement (33) formée dans une paroi périphérique externe du tube en spirale d'entraînement (30) et vissée avec l'au moins une nervure de guidage en spirale (25) du boîtier (20) ;
un manchon de guidage de dosage (60) monté dans le boîtier (20), pouvant tourner en un point de fixation ; et le manchon de guidage de dosage (60) inséré dans l'ensemble de poussée en spirale (2A) et pouvant être commandé pour se relier et tourner avec l'ensemble de poussée en spirale (2A) ; le manchon de guidage de dosage (60) comprenant un corps de manchon de guidage (61) et de multiples boucles à cliquet anti-retour (62) ; le corps de manchon de guidage (61) ayant un trou central de manchon de guidage (63), au moins une portion en spirale (65) formée sur une paroi périphérique interne du trou central (63) du manchon de guidage ; une portion filetée (66) du manchon de guidage formée sur un segment arrière d'une surface périphérique externe du corps (61) du manchon de guidage ; et une portion d'extrémité avant (64) du manchon de guidage formée à une extrémité avant du corps (61) du manchon de guidage ;
les multiples boucles à cliquet anti-retour (62) formées sur une surface externe de la portion d'extrémité avant (64) du manchon de guidage ; lorsque les multiples boucles à cliquet anti-retour (62) sont commandées pour s'engager avec la portion à cliquet à sens unique (23) de le boîtier (20), le manchon de guidage de dosage (60) étant uniquement entraîné pour tourner vers l'avant et dans ledit mode injection ; lorsque les multiples boucles à cliquet anti-retour (62) se désengagent de la portion à cliquet à sens unique (23) du boîtier (20), le manchon de guidage de dosage (60) pouvant tourner vers l'avant ou vers l'arrière et dans ledit mode veille ;
un écrou de commande de volume total (70) monté dans l'ensemble de poussée en spirale (2A), étant capable de se déplacer linéairement le long de l'axe central (A), et vissé avec la portion filetée du manchon de guidage (66) au niveau d'un segment arrière du manchon de guidage de dosage (60) ;
un bouton de tension diagonal (80) monté dans l'extrémité avant du boîtier (20) et étant mobile le long de l'axe central (A) ; le bouton de tension diagonal (80) pouvant être entraîné pour changer une condition d'engagement des boucles à cliquet anti-retour (62) par rapport à la portion à cliquet à sens unique (23) du boîtier (20) et pour convertir ledit mode injection et ledit mode veille ; et
une vis de dosage (90) insérée dans le bouton de tension diagonale (80) et le boîtier (20) et la vis de dosage (90) vissée à l'intérieur du manchon de guidage de dosage (60) ; la vis de dosage (90) ayant un corps de vis (91) ayant une portion filetée (93) et la vis de dosage (90) vissée avec ladite portion en spirale (65) à l'intérieur du manchon de guidage de dosage (60) via la portion filetée (93) ; une extrémité avant de la vis de dosage (90) s'étendant devant le bouton de tension diagonale (80) ; lorsque le mécanisme d'injection (2) est dans le mode injection, le bouton de tension diagonal (80) étant déclenché pour se déplacer vers l'arrière et pour entraîner les multiples boucles à cliquet anti-retour (62) pour venir en prise avec la portion à cliquet à sens unique (23) du boîtier (20), un mouvement en spirale du manchon de guidage de dosage (60) pouvant entraîner la vis de dosage (90) à se déplacer linéairement le long de l'axe central (A) à l'intérieur du boîtier (20) pour effectuer une sortie quantitative du médicament liquide dans le flacon de médicament (3) ; lorsque le mécanisme d'injection (2) dans le mode veille, le bouton de tension diagonal (80) pouvant se déplacer vers l'avant pour faire en sorte que les multiples boucles à cliquet anti-retour (62) se désengagent de la portion à cliquet à sens unique (23) du boîtier (20), la vis de dosage (90) pouvant se déplacer linéairement vers l'arrière et être reçue dans le boîtier (20) le long de l'axe central (A).

2. Seringue selon la revendication 1, **caractérisée en ce que** la seringue comprend un manchon de cartouche (1) ; le manchon de cartouche (1) a un espace de confinement (100) formé à travers et à l'intérieur du manchon de cartouche (1) le long de l'axe central (A) ; le manchon de cartouche (1) est installé de manière amovible sur le flacon de médicament (3), et une extrémité avant du manchon de cartouche (1) est installée de manière amovible sur le composant d'aiguille (3A) ; le manchon de cartouche (1) peut être relié à l'extrémité avant du boîtier (20) du mécanisme d'injection (2) ; et le manchon de cartouche (1) est capable de déclencher le bouton de tension diagonal (80) pour qu'il se déplace vers l'arrière et d'entraîner les multiples boucles à cliquet anti-retour (62) pour qu'elles s'engagent dans la portion à cliquet à sens unique (23) du boîtier (20) et dans ledit mode injection ; et lorsque le manchon de cartouche (1) est détaché de l'extrémité avant du boîtier (20) du mécanisme d'injection (2), le bouton de tension diagonal (80) est capable de se déplacer vers l'avant pour faire en sorte que les multiples boucles à cliquet anti-retour (62) se désengagent de la portion à cliquet à sens unique (23) du boîtier (20) et dans ledit mode veille.

3. Seringue selon la revendication 2, **caractérisée en ce que** le manchon de cartouche (1) comprend un corps de cartouche (10) ; le corps de cartouche (10) a au moins une fenêtre de visualisation (14) formée dans une paroi périphérique du corps de cartouche (10) ; une portion de liaison (12) est formée à une extrémité arrière du corps de cartouche (10) ; une bride de positionnement (13) est formée sur la paroi périphérique du corps de cartouche (10) et située à une extrémité avant de la portion de la liaison (12) ; de multiples saillies d'engagement (15) sont formées sur une surface périphérique externe de la portion de la liaison (12) ; l'extrémité avant du boîtier (20) est une portion de la liaison du manchon de cartouche (21) ; de multiples rainures d'engagement en forme de L (211) sont formées dans une paroi périphérique interne de la portion de la liaison du manchon de cartouche (21), chaque rainure d'engagement en forme de L (211) comprend une section de rainure droite parallèle à l'axe central (A) et une section transversale reliée à une extrémité arrière de la section de rainure droite ; lorsque la portion de la liaison (12) à l'extrémité arrière du manchon de cartouche (1) est inséré dans la portion de la liaison du manchon de cartouche (21) à l'extrémité avant du boîtier (20), chaque saillie d'engagement (15) est capable de coulisser le long de la section de rainure droite de l'une respective des rainures d'engagement en forme de L (211) et de se transformer en une extrémité de la section transversale de l'une respective des rainures d'engagement en forme de L (211) pour verrouiller le manchon de cartouche (1) à l'extrémité avant du boîtier (20).

4. Seringue selon la revendication 1, **caractérisée en ce qu'**une bague de guidage (22) est formée dans l'extrémité avant du boîtier (20) ; une surface périphérique externe de la bague de guidage (22) est reliée à la paroi périphérique de l'espace mobile (200) par l'intermédiaire de multiples bras (24) ; un espacement est formé entre la bague de guidage (22) et la paroi périphérique de l'espace mobile (200) ; au moins un bloc de guidage (221) est formé sur une paroi annulaire interne de la bague de guidage (22) ; le corps de vis (91) de la vis de dosage (90) comporte au moins une rainure de guidage axiale (94) traversant la portion filetée (93) et étant parallèle à l'axe central (A) ; ladite rainure de guidage axiale (94) s'engage avec ledit bloc de guidage (221) à l'intérieur de la bague de guidage (22) du boîtier (20).

5. Seringue selon la revendication 4, **caractérisée en ce que**, chaque boucle à cliquet anti-retour (62) a une aile (621) faisant saillie vers l'avant et disposée au niveau d'une face d'extrémité avant de la boucle à cliquet anti-retour (62) ; le bouton de tension diagonal (80) a une surface conique (82) venant en butée contre l'aile (621) de chaque boucle à cliquet anti-retour (62) pour permettre à la surface conique (82) du bouton de tension diagonal (80) de pousser la boucle à cliquet anti-retour (62) pour qu'il s'engager avec la portion à cliquet à sens unique (23) du boîtier (20) via l'aile (621).

6. Seringue selon la revendication 5, **caractérisée en ce que** la seringue comprend un manchon de cartouche (1) ; le manchon de cartouche (1) a un espace de confinement (100) formé à travers et à l'intérieur du manchon de cartouche (1) le long de l'axe central (A) ; le manchon de cartouche (1) est installé de manière amovible sur le flacon de médicament (3), et une extrémité avant du manchon de cartouche (1) est installée de manière amovible sur le composant d'aiguille (3A) ; le manchon de cartouche (1) peut être relié à l'extrémité avant du boîtier (20) du mécanisme d'injection (2) ; et le manchon de cartouche (1) est capable de déclencher le bouton de tension diagonal (80) pour qu'il se déplace vers l'arrière et d'entraîner les multiples boucles à cliquet anti-retour (62) pour qu'elles s'engagent dans la portion à cliquet à sens unique (23) du boîtier (20) et dans ledit mode injection ; et lorsque le manchon de cartouche (1) est détaché de l'extrémité avant du boîtier (20) du mécanisme d'injection (2), le bouton de tension diagonal (80) est capable de se déplacer vers l'avant pour faire en sorte que les multiples boucles à cliquet anti-retour (62) se désengagent de la portion à cliquet à sens unique (23) du boîtier (20) et dans ledit mode veille.

7. Seringue selon la revendication 6, **caractérisée en ce que**,
le manchon de cartouche (1) comprend un corps de cartouche (10) ; le corps de cartouche (10) a au moins une fenêtre de visualisation (14) formée dans une paroi périphérique du corps de cartouche (10) ; une portion de liaison (12) est formée à une extrémité arrière du corps de cartouche (10) ; une bride de positionnement (13) est formée sur la paroi périphérique du corps de cartouche (10) et située à une extrémité avant de la portion de la liaison (12) ; de multiples saillies d'engagement (15) sont formées sur une surface périphérique externe de la portion de la liaison (12) ; l'extrémité avant du boîtier (20) est une portion de la liaison du manchon de cartouche (21) ; de multiples rainures d'engagement en forme de L (211) sont formées dans une paroi périphérique interne de la portion de la liaison du manchon de cartouche (21), chaque rainure d'engagement en forme de L (211) comprend une section de rainure droite parallèle à l'axe central (A) et une section transversale reliée à une extrémité arrière de la section de rainure droite ; lorsque la portion de la liaison (12) à l'extrémité arrière du manchon de cartouche (1) est inséré dans la portion de la liaison du manchon de cartouche (21) à l'extrémité avant du boîtier (20), chaque saillie d'engagement (15) est capable de coulisser le long de la section de rainure droite de l'une respective des rainures d'engagement en forme de L (211) et de se transformer en une extrémité de la section transversale de l'une respective des rainures d'engagement en forme de L (211) pour verrouiller le manchon de cartouche (1) à l'extrémité avant du boîtier (20).

8. Seringue selon l'une quelconque des revendications 1 à 7, **caractérisée en ce que** l'ensemble de poussée en spirale (2A) comprend un manchon d'embrayage (40) et un bouton de sortie (50), dans laquelle :
le tube en spirale d'entraînement (30) a un canal de tube en spirale (31) formé à travers le tube en spirale d'entraînement (30) le long de l'axe central (A), une surface incurvée de guidage (32) formée au niveau d'un segment avant du canal de tube en spirale (31) et convergeant vers l'avant le long de l'axe central (A), et une portion d'extrémité arrière de tube en spirale (34) disposée à une extrémité arrière du tube en spirale d'entraînement (30) ; la portion d'extrémité arrière de tube en spirale (34) s'étend vers l'extérieur de l'extrémité arrière du boîtier (20) et est bloquée par l'extrémité arrière du boîtier (20) ;
le manchon d'embrayage (40) est monté dans le canal de tube en spirale (31) du tube en spirale d'entraînement (30) et peut se déplacer linéairement par rapport au tube en spirale d'entraînement (30) ; le manchon d'embrayage (40) comporte un corps de manchon (41), de multiples griffes de verrouillage flexibles (42) formées à une extrémité avant du corps de manchon (41) et une portion d'extrémité de la liaison (43) disposée à une extrémité arrière du corps de manchon (41) ; un canal de manchon (44) est formé dans le corps de manchon (41) et s'étend le long de l'axe central (A) ; au moins une portion de rainure de guidage de manchon (45) est formée sur une paroi périphérique du canal de manchon (44) et est parallèle à l'axe central (A) ; les multiples griffes de verrouillage flexibles (42) ont une flexibilité pour la dilatation et la contraction radiales ; lorsque le manchon d'embrayage (40) est déplacé vers l'avant par rapport au tube hélicoïdal d'entraînement (30), les multiples griffes de verrouillage flexibles (42) sont en butée contre la surface incurvée de guidage (32) à une extrémité avant du tube hélicoïdal d'entraînement (30) et se contractent radialement ; et les multiples griffes de verrouillage flexibles (42) sont capables de rebondir vers l'arrière jusqu'à une position d'origine le long de la surface incurvée de guidage (32) et de se dilater ;
le bouton de sortie (50) est disposé à l'extérieur derrière l'extrémité arrière du boîtier (20) et est relié à la portion d'extrémité de la liaison (43) à une extrémité arrière du manchon d'embrayage (40) ;
le manchon de guidage de dosage (60) est inséré dans le tube en spirale d'entraînement (30) et le manchon d'embrayage (40) et peut être serré par les multiples griffes de verrouillage flexibles (42) du manchon d'embrayage (40) ;
l'écrou de commande de volume total (70) a au moins une nervure de bride (71) formée sur une surface périphérique externe de l'écrou de commande de volume total (70) et s'engageant avec ladite au moins une portion de rainure de guidage de manchon (45) du manchon d'embrayage (40).

9. Seringue selon la revendication 8, **caractérisée en ce que** chacune des griffes de verrouillage flexibles (42) a une surface de verrouillage (421) formée au niveau d'une surface interne de la griffe de verrouillage flexible (42) ;
la surface de verrouillage (421) de chacune des griffes de verrouillage flexibles (42) a de multiples bandes droites s'étendant le long d'une direction avant-arrière ; le manchon de guidage de dosage (60) a une surface d'engagement (67) formée sur la surface périphérique externe, située entre la portion d'extrémité avant de manchon de guidage (64) et la portion filetée de manchon de guidage (66), du corps de manchon de guidage (61) ; la surface d'engagement (67) a de multiples bandes droites parallèles à l'axe central (A) et correspondant aux bandes droites des surfaces de verrouillage (421) des griffes de verrouillage flexibles (42) pour l'engagement.

10. Seringue selon la revendication 8, **caractérisée en ce que** le boîtier (20) a une fenêtre d'observation d'échelle (26) formée au niveau d'une section arrière d'une paroi périphérique externe du boîtier (20) ; le tube en spirale d'entraînement (30) a une portion d'échelle en spirale (35) formée sur la paroi périphérique externe du tube en spirale d'entraînement (30) et étant parallèle à la rainure de guidage en spirale d'entraînement (33) ; la portion d'échelle en spirale (35) a de multiples lignes d'échelle pouvant être observées à travers la fenêtre d'observation d'échelle (26) du boîtier (20).
